Europäisches Patentamt

European Patent Office

Office européen des brevets

(19)

(11) **EP 1 577 822 A1**

(12) **EUROPEAN PATENT APPLICATION**

(43) Date of publication:
**21.09.2005 Bulletin 2005/38**

(51) Int Cl.$^7$: **G06F 19/00**

(21) Application number: **04290701.4**

(22) Date of filing: **15.03.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL LT LV MK**<br><br>(71) Applicant: **Avant-Garde Material Simulation SARL<br>78400 Chatou (FR)** | (72) Inventor: **Neumann, Marcus A.,<br>c/o Avant-garde Materials<br>78400 Chatou (FR)**<br><br>(74) Representative: **Betten & Resch<br>Patentanwälte,<br>Theatinerstrasse 8<br>80333 München (DE)** |

(54) **Method for energy ranking of molecular crystals using DFT calculations and empirical van der Waals potentials**

(57) The invention refers to a method for the accurate determination of van der Waals parameters for high-precision determination of crystal structures and/or energies, comprising the steps of: numerically simulating at least one crystal structure based on density functional theory (DFT) calculations combined with a potential energy term representing van der Waals interactions; providing reference data containing accurate information about said at least one crystal structure; defining a deviation function (F) quantifying a deviation between said reference data and said at least one simulated crystal structure; fitting at least one parameter of said van der Waals potential term in such a way as to minimize said deviation function (F); and obtaining the accurate van der Waals parameters from the best fit. The invention furthermore deals with a hybrid method for the accurate determination of crystal structures and/or energies based on such a parameter determination as well as the general application of such a hybrid method to the energy ranking of polymorphic crystal structures.

Fig. 10

## Description

### 1 Introduction and industrial context

The present invention refers to a method for the accurate determination of van der Waals parameters used in conjunction with density functional theory calculations for high-precision determination of crystal structures and/or energies, and based on the thus determined accurate parameters, a method for the accurate determination of crystal structures and/or energies, in particular in view of the energy ranking of polymorphic crystal structures.

The physical and chemical properties of molecular compounds in the crystalline state strongly depend on the precise nature of the molecular arrangement. For most molecules, several crystal polymorphs can be observed experimentally that may differ significantly with respect to their density, hardness, crystal habit, colour, solubility, dissolution rate and other important properties. Therefore, the characterization and the control of polymorphism are of prime importance for various industrial applications such as the production of explosives, pigments and food products. In the pharmaceuticals industry, strict regulatory rules apply to make sure that only well-defined polymorphic forms are brought to market, since changes of the solubility and the dissolution rate affect the bioavailability of a drug compound. One particular problem for the pharmaceuticals industry is the fact that the most stable polymorph may have very unfavourable crystallization kinetics. It may therefore happen that the most stable polymorph appears accidentally many years after the first crystallization experiments, at a point when clinical trials and production scale-up have already been carried out for a meta-stable polymorph. Once the production site has been contaminated with the most stable polymorph, the controlled crystallization of the desired polymorph may turn out to be impossible. This problem, known as the phenomenon of disappearing polymorphs, may cause serious delays and losses of revenue [Dunitz & Bernstein, 1995].

The reasons outlined above have triggered a substantial amount of research in the field of *in silico* polymorph screening, commonly called polymorph prediction, a technique aiming at the prediction of the experimentally accessible polymorphs of a given molecule by computer simulation. The state-of-the-art is illustrated by the results of two blind tests organized by the Cambridge Crystallographic Data Center [Lommerse 2000; Motherwell 2002]. In silico polymorph prediction can be roughly divided into three steps: structure generation, energy ranking and the prediction of crystallization kinetics. Structure generation with a tool like Accelrys' Polymorph Predictor [Accelrys 2004] is usually quite reliable for rigid and slightly flexible molecules. Energy ranking, on the contrary, remains an important challenge. Many different low energy crystal structures are typically found in an energy window of 1 kcal/mol and the current accuracy of lattice energy calculations is not high enough to allow for the identification of the most stable polymorphs. The prediction of crystallization kinetics, i.e. the identification of the crystal polymorph that is the easiest to crystallize, is completely beyond reach.

The current inability to pin-point the experimentally observable crystal structures in a list of generated crystal structures is a serious drawback for industrial applications. Since different polymorphs, even if close in energy, may have very different properties, in silico polymorph prediction is virtually useless as long as it remains impossible to identify those polymorphs that can be crystallized. In the past, *in silico* polymorph screening has sometimes been used for crystal structure solution in conjunction with low quality powder diffraction data, but this approach has now been replaced by more powerful methods for direct crystal structure solution from powder diffraction data.

In this document we describe a new method for accurate lattice energy calculations that will, in general, allow for the identification of the most stable crystal polymorph within a list of candidates. The new method will have a strong impact on the application of *in silico* polymorph screening to industrial problems. Having predicted the most stable polymorph by computer simulation, it will virtually always be possible to find the right conditions to crystallize it. This means that for every molecule, even if it has never been synthesized, one obtainable crystal polymorph with its physical and chemical properties can be identified by computer simulation. It will thus become possible to use computational tools in order to design new materials with the desired colour (pigments), solubility (pharmaceuticals), hyperpolarisability (laser technology) and other wanted features. In the pharmaceuticals area, the new method will be used to identify and crystallize the most stable polymorphic form, thus avoiding the problem of disappearing polymorphs.

### 2 Prior art

Current energy calculations can be roughly divided into three categories: ab initio calculations, semi-empirical calculations and force field calculations (see [Wimmer 2004] for a basic introduction and [Leach 2001] for details):

Ab initio calculations are the most accurate and the most CPU-time consuming. They take into account the quantum nature of the electronic movement around the atomic nuclei. To calculate the energy for a given set of atomic positions, it is necessary so solve Schodinger's equation for the electronic motion. The analytical solution of Schödinger's equation is only possible in the simplest cases (hydrogen atom, etc). In general, the solution of Schödinger's equation involves a certain number of approximations and is carried out numerically. According to the nature of the approximations, *ab initio* calculations can be subdivided into three big families: Hartree-Fock (HF) calculations, Density Functional Theory (DFT) calculations and Quantum Monte Carlo (QMC) simulations [Foulkes et al 2001].

Basic HF calculations are not very accurate because of the neglect of electron correlation. However, augmented by perturbation theory or configuration interaction calculations, HF calculations offer a very high accuracy. In theory, the accuracy of this type of calculation is only limited by the available CPU resources. In practice, the CPU time requirements increase very rapidly with the system size (= number of atoms and electrons) and they are therefore inappropriate for the energy ranking of molecular crystals.

In DFT calculations, an approximation is used for the treatment of electron correlation effects. At comparable accuracy, DFT calculations are usually faster than HF calculations and scale more favourably with system size. DFT calculations on crystal structures can be carried out on a state-of-the-art PC and offer satisfying results for framework structures (zeolithes, etc) and ionic crystals. However, because of the approximate treatment of electron correlation effects, the accuracy of DFT calculations is limited. In particular, the long range Van der Waals (VdW) interactions (also called dispersive interactions) between neutral (or charged) atoms are not taken into account. Van der Waals interactions play an important role in molecular crystals, and pure DFT calculations for molecular crystals are therefore not very accurate.

QMC simulations are highly accurate but require very long calculation times. They are therefore limited to systems containing no more than a few atoms.

Semi-empirical calculations are related to *ab initio* calculations. They can be derived by introducing further approximations and adjustable parameters. The adjustable parameters need to be calibrated with respect to experimental data or high level ab initio calculations. Semi-empirical methods are currently not accurate enough for the accurate energy ranking of molecular crystals.

Force fields are purely empirical in nature and use a set of functional forms and parameters to describe the potential energy as a function of the atomic coordinates. If a force field is carefully parameterized for a particular molecule or a class of related molecules, force field calculations can turn out to be fairly accurate and are sometimes appropriate for the accurate energy ranking of simple molecular crystals. For most molecules of industrial interest, however, well parameterized force fields are not readily available. Force field parameters are generally adjusted for a limited set of rather small molecules and it is assumed that they can be transferred to larger molecule. This parameter transfer induces energy errors that are incompatible with the needs of in silico polymorph screening. In addition, today's force fields perform poorly for systems with strong electrostatic interactions such as molecular salts and zwitterions (e. g. glycine) that play an important role in pharmaceutical applications.

In summary, it can be said that - within the current memory and CPU time constraints - none of the methods mentioned above offers the accuracy required for in silico polymorph screening.

In recent years, several attempts have been made to increase the accuracy of DFT calculations by adding empirical potential energy functions that model the long range dispersive interactions [Elstner et al 2003; Wu and Yang 2002; Liu et al 2001; Elstner et al 2001]. In all these approaches, an additional potential energy term of the following type is used:

$$E_{disp} = \sum_{A,B} - f_{A,B}(r_{A,B}) \frac{C_{6,A,B}}{r_{A,B}^6} \qquad \text{(Eq. 2.a)}$$

Here A and B run over all pairs of interacting atoms, $r_{A,B}$ is the distance between the interacting atoms, $f_{A,B}$ is a damping function and $C_{6,A,B}$ is a coefficient that characterizes the strengths of the interaction. The $-C_6/r^6$ term captures the well known asymptotic behaviour of dispersive interactions at large interatomic distances. To avoid the unrealistic divergence of the empirical potentials at short interatomic distances, a damping functions $f_{A,B}$ is used. The $C_{6,A,B}$ interaction coefficients can be determined from experimental data or high level *ab initio* calculations.

From now on, we will call a method that combines DFT calculations with empirical Van der Waals potentials a hybrid method. In the scientific literature [Elstner et al 2003; Wu and Yang 2002; Liu et al 2001; Elstner et al 2001 the accuracy of the existing hybrid methods has been assessed by comparison with high level HF calculations. Disagreements seem to be of the order of 1 kcal/mol, which is far too much for the accurate energy ranking of crystal structures. The published methods have been applied to rare-gas diatomic molecules, the stacking of base pairs, polyalanines conformation stabilities and protein modelling.

In the hybrid method proposed in the prior art, only the $C_{6,A,B}$ coefficient is numerically adjusted based on reference data which describe long-range molecular interactions, taking advantage of the fact that on a large scale, the interaction between molecules is dominated by a van der Waals potential, whereas contributions expressed by DFT calculations are negligible. As a consequence, such calculations are unsuitable for numerically simulating crystal structures, i.e. molecular arrangements on a geometrical scale at which DFT contributions and van der Waals contributions have the same order of magnitude.

It is therefore an object of the invention to propose a method for the accurate determination of van der Waals parameters which is suitable for the high-precision determination of crystal structures and/or energies.

According to the invention, this object is achieved by a method as defined in claim 1.

Advantageous embodiments are defined in dependent claims 2 to 9.

A particularly efficient numerical optimization method using an advantageous crystal coordinate system is defined in claim 10.

A general advantageous method for the energy ranking of polymorphic crystal structures based on a hybrid method is defined in claim 11.

In this document we describe an improvement of the hybrid approach that makes it suitable for the accurate energy ranking of crystal structures. At present, the scientific community has not realized that for highly accurate calculations the careful adjustment of the damping function f is as important as the determination of appropriate $C_6$ coefficients. In addition, it has not been realized that hybrid methods with properly adjusted empirical parameters offers the accuracy required for the accurate energy ranking of crystal structures in the context of *in silico* polymorph screening.

It is important to note that the term 'hybrid method' is often employed in a different context. To study complex phenomena such as protein ligand interactions, complex systems are often subdivided into two subsystems. For instance, the smaller subsystem may consist of the active site of the protein and a ligand, while the larger subsystem contains the backbone of the protein and the solvent. For the smaller subsystem, the potential energy is determined by high level ab initio calculations, while a force field is used to describe the interactions between the two subsystems and within the larger subsystem. The case just described is different from the hybrid method described in this document, where both the high level DFT calculations and the empirical potential energy terms apply to <u>all</u> atoms in the system.

The hybrid method described in this document provides the potential energy as a function of the unit cell parameters and the atomic positions in the unit cell. The crystal structures observed in nature approximately correspond to local minima of the potential energy as a function of the structural parameters. Lattice energy minimization, that is the determination of the structural parameters that correspond to the lowest value of the potential energy, is therefore a key step for the accurate energy ranking of crystal structures. As we will see later, lattice energy minimization is also a key step in the refinement of the empirical parameters used with the hybrid method. Since energy calculations with the hybrid method are very time consuming, a new efficient lattice energy minimization algorithm for molecular crystals has been developed and is described in this document. We therefore briefly review the state of the art in the field of lattice energy minimization.

In principle, lattice energy minimization is nothing else but the minimization of a function with respect to its variables. Seen from this angle, lattice energy minimization is a standard task (see [Press et al 2002] for more information on standard minimization algorithms).

However, the efficiency of the lattice energy minimization procedure is strongly related to the choice of the coordinate system used to describe the crystal structure. Depending on the coordinate system, the potential energy surface around the minimum is more or less harmonic and most optimization algorithms work best on harmonic potential energy surfaces.

A straight forward choice is to define a crystal in terms of its space group, its lattice parameters (or direction cosines or other related variables) and the positions (in fractional or Cartesian coordinates) of the atoms in the asymmetric unit. The lattice energy minimization algorithms in DFT codes such as VASP [Kresse and Hafner 1993 & 1994, Kresse and Furthmüller 1996 & 1996b, Kresse and Jouber 1999] and CASTEP [Milman et al 2000] work along these lines. Fractional/Cartesian coordinates are an appropriate choice for many inorganic crystals, but they turn out to be very inefficient for molecular crystals. For isolated molecules, it has been shown that so called internal delocalized coordinates [Baker 1997; Baker et al 1996; ] perform significantly better than Cartesian coordinates. A similar approach has been shown to work for crystal structures of 3D covalently bonded networks [Andzelm et al 2001] and has been implemented in the DFT code DMol3 [Delley 1990; Delley 2000]. However, the unit cell was not allowed to vary and the approach has not been combined with space group symmetry. Details about the implementation are not readily available, but it is known that the program can also cope with molecular crystals. This may be achieved by the introduction of fictive additional bonds between molecules to create a 3D bonded network. The use of delocalized internal coordinates for the optimization of periodic systems with a 3D network of covalent bonds has also been reported by a second group [Kudin et al 2001]. In this case, the unit cell is allowed to vary, but once again the approach is not combined with space group symmetry (only the point group of the unit cell is taken into account). Molecular crystals without a 3D network of covalent bonds are dealt with by introducing additional bonds, for instance the hydrogen bonds between molecules.

Both of the above mentioned generalizations of the concept of delocalized internal coordinates to the case of molecular crystals have the disadvantage that the introduction of additional bonds is somewhat arbitrary and that there is no clear distinction between intramolecular and intermolecular degrees of freedom. In general, intramolecular interactions are much stronger than intermolecular interactions, and it is therefore desirable to decouple the intramolecular and the intermolecular degrees of freedom. In addition, both generalizations are not combined with a full treatment of space group symmetry, thus increasing significantly the number of parameters to be refined.

In this document, we describe a coordinate system based on internal delocalized coordinates (for intramolecular degrees of freedom), whole molecule translations and rotations and unit cell changes. We also show how this coordinate system can be combined with space-group symmetry. The new coordinate system is the 'natural choice' for molecular crystals, but has, according to our knowledge, never been implemented. Probably because of the fairly complicated coordinate transformations that are involved.

### 3 Main embodiments of the invention

### 3.1 A highly accurate hybrid method for lattice energy calculations

The invention provides a hybrid method for the calculation of lattice energies and forces (= energy derivatives) in molecular crystals that is - for given CPU time requirements and system size - more accurate than any other method currently available.

The invention also provides a recipe for the determination of the empirical parameters used with the hybrid method. The accuracy of the hybrid method strongly depends on the refinement of appropriate empirical parameters.

### 3.2 A method for the efficient lattice energy minimization of molecular crystals

As part of the invention, a more efficient method for the crystal structure optimization of molecular crystals has been developed.

### 3.3 A method for the accurate energy ranking of molecular crystals

The invention provides a method that is suitable, unlike any other method, for the reliable energy ranking of the various polymorphic crystal structures of one and the same molecule. Accurate energy ranking is essential in the context of in silico polymorph screening.

These embodiments are closely linked and can be combined with each other.

### 4 Detailed description of preferred embodiments

**Preferred embodiments of the invention will be described in detail with reference to the accompanying drawings, in which:**

Fig. 1 shows a schematic representation of the interaction energy between two atoms, illustrating that to yield the correct interaction energy, the DFT calculation and the additional VdW energy need to be complementary.

Fig. 2 shows the distance dependence of the empirical pair potential calculated for the $\gamma$-polymorph of $N_2$.

Fig. 3 illustrates the influence of the form factor n by showing $f_{A,B}(r)*r^{-6}$ for three different values of the form factor with $r_{A,B}=3.0$. The smaller the form factor, the harder the transition from the long range $r^6$ part to the constant short range part.

Fig. 4 shows internal coordinates and Cartesian coordinates for water.

Fig. 5 shows contour plots of the potential energy surface for different definitions of the dimensionless parameters $q_1$ and $q_2$, wherein contour lines represent 10 kcal/mol intervals.

Fig. 6 shows degrees of freedom describing atomic displacements in the unit cell: 1) Whole molecule translations, 2) whole molecule rotations, 3) intramolecular coordinate changes, the two molecules in the unit cell being symmetry related.

Fig. 7 shows the molecular centres following the deformation of the lattice, while the molecular geometry and the molecular orientation remain unchanged.

Fig. 8 illustrates the definition of a bond length, a bond angle and a torsion angle.

Fig. 9 shows three atoms on a straight line.

Fig. 10 shows a flow diagram for parameter refinement.

Fig. 11 shows the test set of molecules used for the energy ranking study, comprising from left to right: ethane, ethylene, acetylene, methanol, acetic acid, urea.

Fig. 12 compares the energy ranking of the Polymorph Predictor (UFF with Qeq charges) to the energy ranking obtained with the hybrid method according to the invention.

Fig. 13 shows the three most stable crystal structures of acetylene found with the hybrid method according to the invention; for rank 1 and rank 3, a superposition with the experimental crystal structure is presented.

Fig. 14 shows a comparison of the experimental and calculated crystal structures of $\alpha$-$N_2$ (left) and $\gamma$-$N_2$ (right) for three different calculations, each of the 6 graphics being a superposition of two crystal structures.

Fig. 15 shows a superposition of the calculated and the experimental crystal structure of $\varepsilon$- $N_2$.

Fig. 16 shows the relative enthalpy as a function of pressure for $\alpha$-$N_2$, $\gamma$-$N_2$ and $\varepsilon$-$N_2$.

### 4.1 A highly accurate hybrid method for lattice energy calculations

Lattice energies are calculated by a combination of high level DFT calculations and additional empirical potentials that model long range dispersive interactions. To achieve optimum complementarily between the DFT part and the empirical part of the calculations, the hybrid method is used to calculate measurable properties and some or all of the empirical parameters are adjusted to reproduce experimental data. The DFT part may involve certain options and all choices have to be made such that best complementarity with the empirical potentials is obtained.

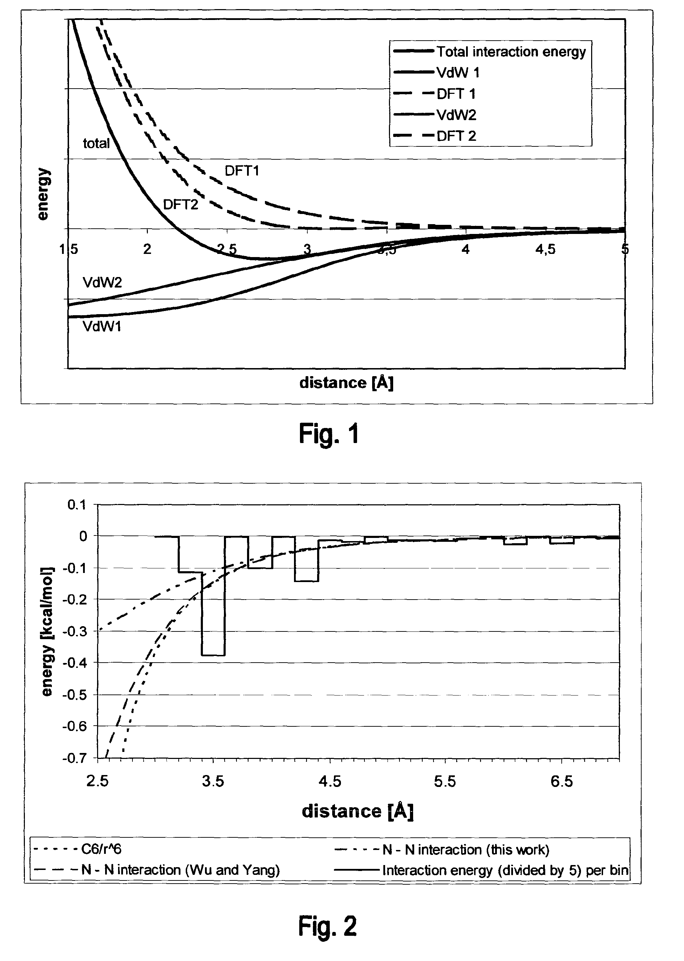

Let us consider the interaction between two neutral atoms (See Fig. 1). Fig. 1 shows a schematic representation of the interaction energy between two atoms, illustrating that to yield the correct interaction energy, the DFT calculation and the additional VdW energy need to be complementary. At large interatomic distances, the contribution of the DFT calculation to the total energy is zero, while the additional empirical VdW potentials follow a $C_6/r^6$-dependence. Since the DFT calculation does not contribute to the total energy, the $C_6$ coefficients can be determined independently, i.e. they do not depend on the DFT calculation. At short interatomic distances, there may be some contribution from the empirical potentials, but the total interaction energy is dominated by the DFT contribution and the contribution from the empirical potentials is negligible.

At intermediate distances, the situation is very different. Both, the DFT contribution and the VdW contribution are equally important, and the correct interaction energy is only obtained if the DFT calculation and the additional empirical potentials are complementary like the two curves DFT1 and VdW1 and the two curves DFT2 and VDW2 in Fig. 1. The contribution of the DFT calculation at intermediate distances depends on various factors such as the basis set and the exchange-correlation functional. As the DFT contribution and the VdW contribution are interrelated, the additional empirical potentials need to be carefully adjusted in the range of intermediate distances. This can be achieved by the appropriate choice of the damping function $f_{A,B}$ (see Eq. 2.a).

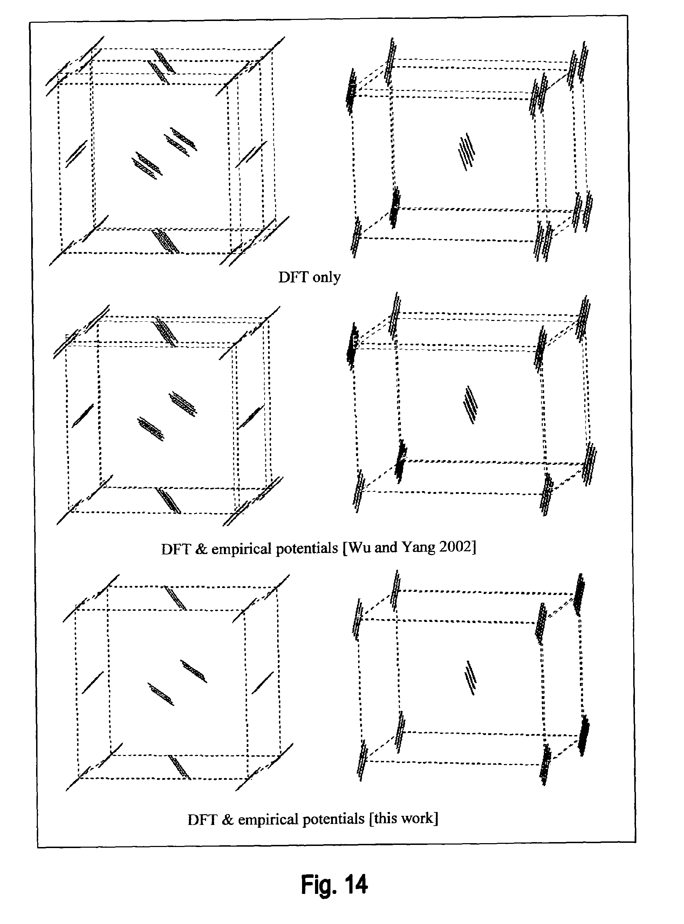

In previous work, only little attention has been paid to the damping function and the parameters involved seem to have been chosen rather ad hoc. This may have been motivated by the fact that there are much more atom pairs at large interatomic distances than at short and intermediate interatomic distances. However, for a given atom, the number of interacting partners increases like $r^2$ with the interatomic distance while the interaction energy decreases like $r^{-6}$. The total interaction energy as a function of the interatomic distance therefore decreases like $r^{-4}$. In other words, most of the interaction energy is concentrated at intermediate atomic distances. This fact is further illustrated by Fig. 2. Fig. 2 shows the distance dependence of the empirical pair potential calculated for the $\gamma$-polymorph of $N_2$. The figure shows the $C_6/r^6$ part of the N-N interaction (dotted curve) and the damped interaction energy (dashed curve) according to Wu and Yang [Wu and Yang 2002]. The dash-dotted curve represents the damped interaction energy that is obtained if the $R_m$ parameter in the damping function of Wu and Yang [Wu and Yang 2002] is adjusted to yield best results in conjunction with the DFT calculations that are described in section 5.2. The solid curve shows a history plot of the VdW interaction energy as a function of the interatomic distance calculated for the $\gamma$-polymorph of $N_2$. The interaction energy is clearly concentrated at short distances and is significantly affected by the damping function. The use of the damping function proposed by Wu and Yang [Wu and Yang 2002] without parameter adjustment would have lead to an overestimation of the total interaction energy (see also section 5.7 and Fig. 14 in particular).

To achieve high accuracy, the adjustment of the damping function is of crucial importance. We have already pointed out that the damping function to use depends on the exact nature of the DFT calculation and more specifically on the choice of the exchange-correlation functional and the basis set. Because of the later dependence, damping functions are probably not transferable between hybrid methods that use localized atomic orbitals and hybrid methods that use 3D periodic basis functions.

In this work we are specifically interested in damping functions for hybrid methods that can cope with 3D periodic boundary conditions. To adjust the damping functions, experimental data are required that can be accurately measured and that are straight forward to calculate. Low temperature crystal structures are ideal for this purpose. At low temperature, the average atomic positions and unit cell parameters measured by a diffraction experiment correspond fairly accurately to the minimum of the potential energy hyper surface, and the minimum energy crystal structure is straight forward to calculate using the hybrid method in conjunction with a lattice energy minimizer. Appropriate damping functions can thus be determined by seeking the best possible agreement between two sets of calculated and experimental crystal structures.

The description of long range dispersive interactions as a sum over isotropic pair potentials is only an approximation. In reality, there are also contributions from many body interactions, i.e. interactions involving more than two atoms. Since we adjust the empirical pair potentials to experimental data, it can be expected that effects such as many body interactions are to a certain extend reflected by the obtained parameters.

Ultimately we are interested in the accurate energy ranking of crystal structures and not in the calculation of accurate crystal structures. It is not obvious that damping functions adjusted toward structural data are also the best possible choice for the comparison of lattice energies. One may argue, however, that crystal structures result from a fine balance between different types of interactions. Since electrostatic interactions are described fairly accurately by DFT calculations, one may conclude that the VdW component of the lattice energy is dealt with accurately if the experimental crystal structures are accurately reproduced. Reliable experimental energetic information is only available for very few compounds. In some cases, the energy ranking of different polymorphs at low temperature is known experimentally and this type of information may be included in the refinement. In addition, one may assume that simple, ball-like molecules with essentially isotropic intermolecular interactions always manage to reach the most stable crystal structure at low temperature. In that case, the experimental low energy crystal structure must be lower in energy than any other structure generated with a tool like Accelrys' Polymorph Predictor [Accelrys 2004]. This type of information may also be included in the refinement.

So far we have focused on the adjustment of the damping function. If enough experimental data is available, one may also refine the $C_6$ coefficients or use more sophisticated descriptions of long range dispersive interactions that may involve anisotropic pair potentials or many body effects.

In an improved embodiment, for a more accurate parameter refinement, the effect of lattice vibrations on the average cell parameters, the average atomic positions and the free lattice energy may be taken into account at the cost of significantly longer calculation times.

Additional empirical potential energy terms (hydrogen bond potentials, conformational energies) unrelated to long range dispersive interactions may be added to the hybrid method to correct for failures of the hybrid method that may have been identifies by the comparison of experimental and calculated low energy crystal structures.

The empirical parameters used with the hybrid method may be adjusted to theoretical rather than experimental data. Theoretical data, for example energies, forces and pressure components, may be calculated for a set of crystal structures using high level HF calculations (with corrections taking into account electron correlation effects) or quantum Monte Carlo techniques. Compared to the use of experimental data, this approach has advantages and disadvantages. On the one hand, theoretical data may be useful in cases where experimental data are not readily available. In addition, theoretical data are not affected by effects such as zero-point vibrations which are difficult to take into account in the refinement procedure. On the other hand, the accuracy of theoretical data is affected by the limitations of the corresponding calculations and there may be significant deviations from reality. Therefore, refinement with respect to experimental data has been preferred in this work.

## 4.2 A method for the efficient lattice energy minimization of molecular crystals

An advantageous feature is the choice of a coordinate system that uses delocalized internal coordinates for the intramolecular degrees of freedom and whole molecule translations and rotations for the intermolecular degrees of freedom. Lattice changes are described in terms of deformations of the starting lattice without any rotational component. Upon lattice changes, the fractional coordinates of the molecular centres (of mass, of volume or other) remain constant and no rotation of the molecules is induced. Space group symmetry is fully taken into account.

The choice of coordinates provides good decoupling of the various degrees of freedom involved. The use of delocalized internal coordinates reflects the chemical connectivity and takes into account the inherent curvilinear nature of intramolecular structure changes. The use of whole molecule translations and rotations allows for whole molecule displacements without strong changes of the intramolecular forces. The treatment of lattice changes described above insures that lattice changes affect as little as possible the net force and the net torque acting on the molecules. Full space group symmetry is required to work with the smallest possible number of degrees of freedom.

The lattice energy and its derivatives are typically calculated using a simple coordinate system (called the fractional coordinate system in this document) based on lattice parameters and fractional atomic coordinates. In comparison to the fractional coordinate system, the natural coordinate system involves time consuming additional coordinate transformations. Therefore, the natural coordinate system is a particularly good choice for lattice energy minimization if the computational cost of the additional transformations is small compared to the computational cost of the avoided energy calculations. This is definitely the case when lattice energies are calculated with the hybrid method.

Lattice changes can be described in many different ways. Some of these changes may involve a rotational component. If such a coordinate change is accompanied by a corresponding rotation of all molecules that compensates for the rotation induced by the lattice change, the situation is equivalent to the one described above.

### 4.3 A method for the accurate energy ranking of molecular crystals

For each crystal in a list of polymorphic crystal structures, the corresponding local minimum on the lattice energy hyper surface can be determined using a lattice energy minimizer in conjunction with a hybrid method that combines DFT calculations and empirical potentials for the description of long range dispersive interactions. The crystal structures are ranked with respect to the lattice energy at the local minima.

To be able to identify the most stable crystal structure in a list of crystal structures with a reasonable amount of certainty, lattice energies need to be calculated with an accuracy that exceeds typical energy differences obtained for the structures in the list. The hybrid method described in 4.1 provides this accuracy, a fact that the scientific community is currently not aware of.

The lattice energy at the minima of the potential energy hyper surface is only an approximate indicator for the relative stability of polymorphic crystal structures. In reality, the relative stability is related to the free lattice energy that depends on the lattice energy at the minimum, the energy levels of the lattice vibrations and the temperature. Even at vanishing temperature, the relative stability is not exactly described by the lattice energy minima alone because of the presence of zero-point vibrations.

A more accurate energy ranking could be obtained if the hybrid method was coupled with a free lattice energy minimizer rather than a lattice energy minimizer. Alternatively, one may calculate a free lattice energy correction after lattice energy minimization. However, because of the vibrational component, the calculation of free lattice energies is significantly more time consuming than the calculation of lattice energies.

Alternatively, in a first step, one may parameterize a force field or a semi-empirical method using the energies and energy derivatives determined with the hybrid method. Using this force field or semi-empirical method, the energy ranking may be carried out in a second step. It is thus possible to avoid direct energy ranking using the hybrid method. Since the hybrid method is fairly memory and CPU time expensive, such a two step approach would help to save CPU time or allow calculations for bigger crystal structures. The increased speed of the calculations would also facilitate the calculation of free lattice energies instead of lattice energies. The high precision force field or semi-empirical method may also be used in other areas such as the prediction of crystal morphologies or the computer simulation of liquids.

### 5 Mathematical details and their application

The knowledge of the potential energy $E_{pot}(c_0, ..., c_{nc}, \vec{v}_1, ... \vec{v}_{nv})$ as a function of the unit cell parameters $c_i$ and the atomic positions $\vec{v}_i$ is the key to the calculation of virtually all physical and chemical properties. The variables $c_i$ and $\vec{v}_i$ will be defined more precisely in section 5.1. Within the framework of the hybrid method, the potential energy $E_{pot}$ is separated into two components, a DFT part and a Van der Waals part:

$$E_{pot} = E_{DFT} + E_{disp} \qquad (Eq.\ 5.a)$$

The calculation of $E_{DFT}(c_0, ..., c_{nc}, \vec{v}_1, ... \vec{v}_{nv})$ and its first derivatives is described in section 5.2, while section 5.3 is devoted to the determination of $E_{disp}(c_0, ..., c_{nc}, \vec{v}_1, ... \vec{v}_{nv})$ and its first derivatives.

In the current embodiment of the invention, $E_{pot}$ is used conjunction with a lattice energy minimizer to determine local minima on the lattice energy hyper surface. For the purpose of lattice energy minimization, the fractional coordinate system $(c_0, ..., c_{nc}, \vec{v}_1, ... \vec{v}_{nv})$ is not the ideal choice. In section 5.4, we describe so called natural coordinate system that is based on lattice changes $\lambda_i$, whole molecule translations $\tau_{i,j}$, whole molecule rotations $\rho_{i,j}$ and delocalized

internal coordinates $\theta_{i,j}$. Here the first index runs over all coordinates in a molecule and the second index specifies the molecule. In section 5.4, we also discuss how the lattice energy and its first derivatives can be obtained in the natural coordinate system. Knowing $E_{pot}(\lambda_0, ..., \lambda_{n\lambda}, \tau_{1,1}, ... \tau_{n\tau1,1}, \rho1,1, ... \rho_{n\rho1,1}, \theta_{1,1}, ... \theta_{n\theta1,1}, \tau_{1,2}, ...)$ and its first derivatives, lattice energy minimization can be carried out easily using slightly adapted standard optimization algorithms such as the conjugate gradient technique or quasi-Newton methods (see [Press et al 2002] for details). Since these techniques are well documented in the scientific literature, they are not further discussed.

The empirical energy term $E_{VDW}$ contains parameters that need to be adjusted to experimental data. The parameter refinement procedure is described in section 5.5 and the application of the hybrid method to the energy ranking of crystal structures is discussed in section 5.6. Sections 5.5 and 5.6 both present a variety of validation results which demonstrate that the hybrid methods, with empirical parameters fitted to low temperature crystal structures, is appropriate for the accurate energy ranking of crystal structures. To further illustrate this fact by a simple example, section 5.7 presents a validation study on the polymorphism of $N_2$.

In this work, we mainly concentrate on the calculation of lattice energies. Many of the known crystal structures of $N_2$ have been measured under high pressure. The pressure dependence can easily be taken into account by calculating the lattice enthalpy rather than the lattice energy, i.e. by adding an enthalpy term $pV_{cell}$ to (Eq. 5.a). Here p is the constant, isotropic pressure and $V_{cell}$ is the unit cell volume. The determination of $V_{cell}$ and its first derivatives is discussed in subsection 5.3.2.

**5.1 Lattice parameters, fractional coordinates and symmetry**

For the purpose of lattice energy calculation, we define the crystal structure in terms of nc unit cell parameters $c_i$ and nv atomic positions $v_i$.

The unit cell is entirely defined by the unit cell lengths a, b, c and the unit cell angles $\alpha$, $\beta$, $\gamma$. The number of independent, variable cell parameters $c_i$ depends on the crystal symmetry, or, to be more precise, on the crystal lattice. The table below presents the definition of the variable cell parameters $c_i$ for the seven crystal systems.

Tab. 5.1.a:

| Relationship between the unit cell parameters a, b, c, $\alpha$, $\beta$, $\gamma$ and the nc variable cell parameters $c_i$ | | | | | | | |
|---|---|---|---|---|---|---|---|
| Crystal system | nc | a = | b = | c = | $\alpha$ = | $\beta$ = | $\gamma$ = |
| triclinic | 6 | c1 | c2 | c3 | c4 | c5 | c6 |
| monoclinic | 4 | c1 | c2 | c3 | 90° | c4 | 90° |
| orthorhombic | 3 | c1 | c2 | c3 | 90° | 90° | 90° |
| tetragonal | 2 | c1 | c1 | c2 | 90° | 90° | 90° |
| hexagonal | 2 | c1 | c1 | c2 | 90° | 90° | 120° |
| trigonal | 2 | c1 | c1 | c1 | c2 | c2 | c2 |
| cubic | 1 | c1 | c1 | c1 | 90° | 90° | 90° |

The unit cell parameters defme the lengths of and the angles between the unit cell vectors $\vec{a}$, $\vec{b}$ and $\vec{c}$. For the orientation of the unit cell with respect to an external Cartesian coordinate system we use the convention that $\vec{a}$ is parallel to the x-axis while $\vec{b}$ lies in the x-y plane and has a positive by component. With these definitions, the unit cell vectors are related to the unit cell parameters as follows:

$$\vec{a} = \begin{pmatrix} a_x \\ a_y \\ a_z \end{pmatrix} = \begin{pmatrix} a \\ 0 \\ 0 \end{pmatrix} \qquad \text{(Eq. 5.1.a)}$$

$$\vec{b} = \begin{pmatrix} b_x \\ b_y \\ b_z \end{pmatrix} = \begin{pmatrix} b * \cos(\pi / 180.0 * \gamma) \\ b * \sin(\pi / 180.0 * \gamma) \\ 0 \end{pmatrix} \qquad \text{(Eq. 5.1.b)}$$

$$\vec{c} = \begin{pmatrix} c_x \\ c_y \\ c_z \end{pmatrix} = \begin{pmatrix} c * \cos(\pi / 180.0 * \beta) \\ (b * c * \cos(\pi / 180.0 * \alpha) - b_x c_x) / b_y \\ \sqrt{c^2 - c_x^2 - c_y^2} \end{pmatrix} \qquad \text{(Eq. 5.1.c)}$$

The lattice vectors $\vec{a}$, $\vec{b}$ and $\vec{c}$ are the columns of the transformation matrix $\bar{L}$ from fractional atomic coordinates to Cartesian atomic coordinates:

$$\bar{L} = (\vec{a}, \vec{b}, \vec{c}) \qquad \text{(Eq. 5.1.d)}$$

$$\vec{x}_{cart} = \bar{L}\vec{x}_{fract} \qquad \text{(Eq. 5.1.e)}$$

At different occasions, we will require the derivatives $\partial \bar{L} / \partial c_i$ of the transformation matrix $\bar{L}$ with respect to the variable lattice parameters $c_i$. We do not specify these derivatives explicitly, as they are easily obtained for each crystal system by the application of standard derivation rules after the replacement of the unit cell parameters a, b, c, $\alpha$, $\beta$ and $\gamma$ in (Eq. 5.1.a) - (Eq. 5.1.c) by the values and parameters indicated in Tab. 5.1.a.

The positions of the atoms in the asymmetric unit are given by nv vectors $\vec{v}_i$. These coordinate vectors are related to fractional atomic coordinates by the following equation:

$$\vec{x}_{fract,i} = \vec{W}_i \vec{v}_i + \vec{w}_i \qquad \text{(Eq. 5.1.f)}$$

Here $\vec{W}_i$ is a 3x3 matrix and $w_i$ is a vector. For atoms on a general position, $\vec{W}_i$ is the identity matrix and $w_i$ is zero so that $x_{fract,i}$ and $\vec{v}_i$ are identical. For atoms on special positions, the situation is a little bit different. Because of symmetry constraints, atoms on special positions cannot move freely in all three dimensions. There are tree different situations:

1) The atom cannot move at all: We do not require a vector $\vec{v}_i$ to describe its positions.

2) The atom can move along a line: The first column of the matrix $\vec{W}_i$ is a unit vector along this line, completed by two additional directions such that $\vec{W}_i$ is an orthonormal matrix. Only the first component of $\vec{v}_i$ is meaningful, the other two components are always set to zero. $\vec{w}_i$ is a reference position on the line and may be chosen to be the starting position.

3) The atom can move along a plane: The first two column of the matrix $\vec{W}_i$ are orthogonal unit vectors that lie in the plane completed by one additional direction such that $\vec{W}_i$ is an orthonormal matrix. Only the first two components of $\vec{v}_i$ are meaningful, the last component is always set to zero. $\vec{w}_i$ is a reference position on the plane and may be chosen to be the starting position.

Each atom i in the asymmetric unit has $m_i$ symmetry copies in the unit cell. The number of symmetry copies $m_i$ is called the multiplicity. In general, a symmetry operation consists of a 3x3 matrix $\vec{S}$ and a displacement $\vec{s}$. A list of all symmetry operations for each space can be found in the scientific literature [Hahn 2002]. The number of symmetry copies is not the same for all atoms in the asymmetric unit. Atoms on special positions have fewer symmetry copies than atoms on general positions and there are different types of special positions. Each set of symmetry operations contains at least the identity operation. For each atom i, there is a set of $m_i$ symmetry operations that generates all symmetry copies in the unit cell, and the j-th symmetry copy is given by the following expression:

$$\vec{x}'_{fract,i,j} = \overline{S}_{i,j}\vec{x}_{fract,i} + \vec{s}_{i,j} \qquad \text{(Eq. 5.1.g)}$$

A crystal consists of an infinite number of unit cells and $\vec{x}'_{fract,i,j}$ does not necessarily fall into the same unit cell as $\vec{x}_{fract,i}$. However, it is always possible to find a lattice translation $\vec{t}_{hkl}$=(h,k,1) such that $\vec{x}'_{fract,i,j} + \vec{t}_{hkl}$ and $\vec{x}_{fract,i}$ fall into the same unit cell (h, k and I are integer numbers).

## 5.2 Calculation of $E_{DFT}$ and its first derivatives

All DFT calculations are carried out with the VASP program [Kresse and Hafner 1993 & 1994, Kresse and Furthmuller 1996 & 1996b, Kresse and Jouber 1999, VASP 2004] which was purchased from the University of Vienna. Since the VASP program is commercially available and well documented in the accompanying literature, we essential adopt a black box approach with respect to this part of the lattice energy calculation. However, we have to discuss the choice of certain parameters that affect the numerical accuracy of the calculations. We also describe how the energies, pressures and forces computed by VASP can be converted to the equivalent energies and energy derivatives used within the hybrid method.

### 5.2.1 VASP settings

The numerical results of DFT calculations depend on various approximations, in particular on the choice of the exchange-correlation functional and the basis set.
VASP offers a selection of different exchange-correlation functionals. The local density approximation (LDA) and the generalized gradient approximation (GGA) with the revised Perdew-Burke-Ernzerhof functional were discarded right from the start because - even in the absence of additional empirical potentials - they predict unrealistically strong binding energies for simple molecular crystals such as $N_2$ in which Van der Waals interactions play a dominant role. These binding energies result from erroneous interaction energies at short interatomic distances and do not reflect the correct long range behaviour of dispersive interactions. If the Perdew-Burke-Ernzerhof functional or the Perdew-Wang-91 functional is employed, the generalized gradient approximation seems to work well in conjunction with additional empirical potentials. Detailed validation work has so far only been carried out for the Perdew-Wang-91 functional and all results presented in this document were obtained using this functional.
VASP uses a plane wave basis set with ultra-soft pseudopotentials or in conjunction with the projector augmented wave (PAW) method. All results presented in document were obtained with the PAW method which is considered to be less time consuming than the use of ultra-soft pseudopotentials at comparable accuracy. The numerical accuracy of any calculation with VASP strongly depends on the size of the plane wave basis set which is determined by the plane wave energy cut-off $E_{cut}$ and the k-point spacing. Accuracy and CPU time requirements increase with the number of wavefunctions. If the Monkhorst-Pack scheme is used to determine the k-point grid, a single parameter 1 is sufficient to define the k-point spacing (see section 5.5.2 in [VASP 2004]). 1/1 roughly corresponds to the distances between neighbouring k-points measured in $Å^{-1}$. Different parameterizations of the PAW method are supplied with VASP. For example, soft, normal and hard PAW potentials are provided for first row elements which are the main constituents of organic compounds. Soft potentials offer only a limited accuracy but are compatible with a low number of plane wave basis functions. Hard potentials, on the contrary, offer the best possible ultimate accuracy but require a high number of plane wave basis functions. All results presented in this document were obtained with normal PAW potentials.
To choose appropriate values for $E_{cut}$ and 1 as well as an appropriate set of PAW potentials, a series of single point energy calculations with increasing values of $E_{cut}$ and 1 was performed for various crystal packings of acetylene, ethylene, ethane and glycine. Using normal PAW potentials, it was found that the relative lattice energies are converged to within 0.01 kcal/mol per molecule for $E_{cut}$=520eV and 1=15. All results presented in this document were obtained using these values. If hard PAW potentials are used instead of normal PAW potentials, the energy cut-off needs to be increased from 520 eV to 910 to achieve similar convergence. The converged relative lattice energies obtained with normal and hard PAW potentials turned out to agree to within less than 0.01 kcal/mol. In summary, it can be said that relative lattice energies can be obtained with a numerical accuracy of about 0.01 kcal/mol per molecule (tested only for small organic molecules) if VASP is used with normal PAW potentials and $E_{cut}$=520eV and 1=15. It is important to add that this accuracy is only obtained if the k-point grid of each crystal structure has at least two k-points along each direction of the reciprocal lattice. For crystal structures with large unit cells in direct space, additional k-points need to be added to the Monkhorst-Pack grid obtained with 1=15.

### 5.2.2 Transformations

For every energy calculation, VASP creates an OUTCAR file that contains among other data the lattice energy, the forces on all atoms in the unit cell and the pressure tensor. We now discuss the transformations that are required to convert these results to the energy and the energy derivatives used by the hybrid method.

The lattice energy in the OUTCAR file is given in eV, while the energy unit used with the hybrid method is kcal/mol. The following conversion factor is employed:

$$C_{eV->kcal/mol} = 4.339314*10^{-2} \text{ kcal/mol/eV} \qquad \text{(Eq. 5.2.2.a)}$$

The same conversion factor is used to convert forces from eV/Å to kcal/mol/Å and the components of the stress tensor from eV to kcal/mol. For all atoms in the unit cell, the net force $\vec{F}_{cart,i,j}$ (Force on symmetry copy j of atom i in the asymmetric unit) is specified in the OUTCAR file with respect to the external Cartesian coordinate system in which the lattice vectors are defined. The energy derivative $\partial E_{DFT} / \partial \vec{v}_i$ with respect to the atomic positions in the factional coordinate system can be calculated from these forces. If the coordinate vector $\vec{v}_i$ changes by $\Delta \vec{v}_i$, the Cartesian displacement of the j-th symmetry copy of the i-th atom is:

$$\Delta \vec{x}_{cart,i,j} = \overline{L}\,\overline{S}_{i,j}\,\overline{W}_i \Delta \vec{v}_i \qquad \text{(Eq. 5.2.2.b)}$$

The matrices $\overline{L}$, $\overline{S}_{i,j}$ and $\overline{W}_i$ are defined in section 5.1. For small displacements, the energy change is given by the following expression.

$$\Delta E_{DFT} = \Delta \vec{v}_i^{\,T} * \frac{\partial E_{DFT}}{\partial \vec{v}_i} = \sum_{j=1}^{m_i} \Delta \vec{x}_{cart,i,j}^{\,T} * \vec{F}_{cart,i,j} \qquad \text{(Eq. 5.2.2.c)}$$

Inserting (Eq. 5.2.2.b) into (Eq. 5.2.2.c) and taking into account that the equation must hold for all $\Delta v_i$ that are sufficiently small, we obtain:

$$\frac{\partial E_{DFT}}{\partial \vec{v}_i} = \sum_{j=1}^{m_i} \overline{W}_i^{\,T} * \overline{S}_{i,j}^{\,T} * \overline{L}^T * \vec{F}_{cart,i,j} \qquad \text{(Eq. 5.2.2.d)}$$

The stress tensor $\overline{\tau}$ is specified in the VASP OUTCAR file. For small lattice changes, the strain tensor $\overline{\varepsilon}$ and the stress tensor $\overline{\tau}$ are related to the to the total energy change by the following equation:

$$\Delta E = \sum_{i,j=1}^{3} \varepsilon_{i,j}\,\tau_{i;j} \qquad \text{(Eq. 5.2.2.e)}$$

We now express the strain tensor $\overline{\varepsilon}$ in terms of the transformation matrix $\overline{L}$ and its derivatives with respect to the cell parameters $c_i$. If the transformation matrix changes from $\overline{L}$ to $\overline{L}' = \overline{L} + \Delta c_i * \vartheta L / \vartheta c_i$, a point $\vec{x}_{cart}$ in Cartesian coordinates is moved to:

$$\vec{x}'_{cart} = \overline{L}'\overline{L}^{-1} = (\overline{L} + \Delta c_i \frac{\partial \overline{L}}{\partial c_i})\overline{L}^{-1} = \overline{I} + \Delta c_i \frac{\partial \overline{L}}{\partial c_i}\overline{L}^{-1} = \overline{I} + \overline{\varepsilon} \qquad \text{(Eq. 5.2.2.f)}$$

Here $\overline{I}$ is the identity matrix. The last equality defines the strain tensor $\overline{\varepsilon}$. Inserting this definition into (Eq.

5.2.2.e) and taking into account that the energy change is also given by $\Delta E_{DFT} = \Delta c_i * \partial E_{DFT} / \partial c_i$, we obtain:

$$\frac{\partial E_{DFT}}{\partial c_k} = \sum_{i,j=1}^{3} (\frac{\partial \overline{L}}{\partial c_k} \overline{L}^{-1})_{i,j} \tau_{i;j} \qquad \text{(Eq. 5.2.2.g)}$$

### 5.2.3 CPU time reduction

In the case of centered crystal structures (e.g. space group C 2/c), the CPU time requirements can be significantly decreased if the DFT calculations are carried out for the (smaller) reduced cell rather than for the standard unit cell. Further transformations are required in this case to transform the forces and pressures obtained for the reduced cell to the standard unit cell.

Another way to save CPU time is to use the wavefunction from a previous calculation as a starting point for a new energy calculation if the difference of the corresponding crystal structures is small. This approach is commonly used by DFT structure optimization algorithms. For lattice energy calculations with variable unit cell parameters, it has to be taken into account that the basis set obtained for a fixed energy cut-off depends on the unit cell. As the unit cell changes, the plane wave energy levels change as well. It would be very inconvenient to change the basis set each time an energy level crosses the energy cut-off, because basis set changes result in small discontinuities of the lattice energy. Therefore, lattice energy minimizations are carried out with a constant basis set and the wavefunction from a previous calculation can be used as a starting point for the next calculation. However, to calculate reliable lattice energies, it is important to redefine the basis set at the end of the lattice energy optimization and to start a second lattice energy optimization using the new basis set. This procedure needs to be repeated until the energy changes between successive lattice energy optimizations are small enough.

### 5.3 Calculation of $E_{Vdw}$ and its first derivatives

### 5.3.1 Energy

In this work, we use atomic pair potentials $E_{A,B}$ to describe long range dispersive interactions. The total interaction energy $E_{disp}$ per unit cell can be obtained by summing over all relevant atom pairs:

$$E_{disp} = \frac{K}{V_{cell}} + \sum_{A} \sum_{B} \sum_{j=1}^{m_B} \sum_{\substack{h,k,l \\ 0<|\Delta \vec{r}|\leq R_c}} \frac{1}{2} m_A E_{A,B}(|\Delta \vec{r}|) \qquad \text{(Eq. 5.3.1.a)}$$

$$\Delta \vec{r} = \vec{x}_{cart,A} - \vec{x}_{cart,B,j} - \vec{x}_{cart,h,k,l} \qquad \text{(Eq. 5.3.1.b)}$$

$$\vec{x}_{cart,A} = \overline{L} (\overline{S}_{A,0} (\overline{W}_A \vec{v}_A + w_A ) + \vec{s}_{A,0}) \qquad \text{(Eq. 5.3.1.c)}$$

$$\vec{x}_{cart,B,j} = \overline{L} (\overline{S}_{B,j}(\overline{W}_B \vec{v}_B + w_B) + \vec{s}_{B,j}) \qquad \text{(Eq. 5.3.1.d)}$$

$$\vec{x}_{cart,h,k,l} = \overline{L}\begin{pmatrix} h \\ k \\ l \end{pmatrix} \qquad \text{(Eq. 5.3.1.e)}$$

In (Eq. 5.3.1.a), the first two sums both run over all atoms in the asymmetric unit. The third sum covers all $m_B$ symmetry copies of atom B in the unit cell. Finally, the last sum runs over all lattice translations of the i-th symmetry copy of atom B for which the distance $|\vec{\Delta r}|$ with respect to atom A is bigger than zero (-> no self interaction) and smaller than a cut-off radius $R_c$. The interaction energy $E_{A,B}$ of each atom pair is multiplied with the number of symmetry copies $m_A$ of atom A in the asymmetric unit. The factor ½ avoids double counting. The term $K / V_{cell}$ corrects for the systematic underestimation of $E_{disp}$ due to the use of a finite cut-off radius $R_c$ and will be further discussed below. (Eq. 5.3.1.b)- (Eq. 5.3.1.e) define the distance vector $\vec{\Delta r}$. The matrices and vectors used in these equations are defined in section 5.1.

The pair interaction energy $E_{A,B}$ is the product of a damping function $f_{A,B}$, a spline function $g_{A,B}$ and a $C_{6,A,B}/r^6_{A,B}$ term that describes the asymptotic behaviour of long range dispersive interactions:

$$E_{A,B}(r) = -f_{A,B}(r) * \frac{C_{6,A,B}}{r^6} * g(r) \qquad \text{(Eq. 5.3.1.f)}$$

Here we have used r= $|\vec{\Delta r}|$. $C_{6,A,B}$ is a constant that is different for each pair of atom types. The determination of the $C_6$ coefficients is discussed in section 5.5.

For the damping function, we use a generalized version of the damping function employed by Wu and Yang [Wu and Yang 2002]:

$$f_{A,B}(r) = \left(1 - \exp\left[-c\left(\frac{r}{r_{A,B}}\right)^{\frac{3}{n}}\right]\right)^{2n} \qquad \text{(Eq. 5.3.1.g)}$$

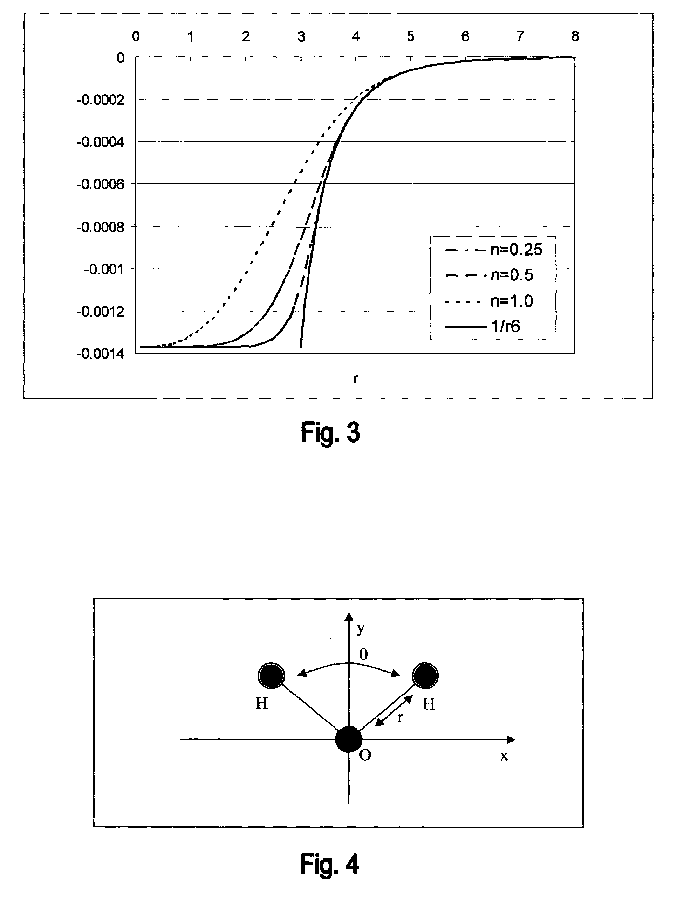

In the paper of Wu and Yang, c is set to 3.54, n is set to 1.0 and the damping radii $r_{A,B}$ are the sum of Van der Waals radii taken from literature [Bondi 1964]. In this work (see section 5.5), we adjust the damping radii $r_{A,B}$ to experimental data. We can therefore choose c=1.0 without loss of generality. In addition, we also adjust the form factor n to experimental data. Fig. 3 illustrates the influence of the form factor n by showing $f_{AB}(r)*r^{-6}$ for three different values of the form factor with $r_{A,B}$=3.0. The smaller the form factor, the harder the transition from the long range $r^6$ part to the constant short range part. As illustrated by Fig. 3, n determines the hardness of the damping function. For the sake of simplicity, we use the same form factor for all atoms pairs. The generalization to atom type dependant form factors is straight-forward.

The spline function in (Eq. 5.3.1.f) is required to avoid discontinuities. Without the spline function, the total dispersion energy $E_{disp}$ would change discontinuously each time that the distance of an atom pair crosses the cut-off distance $R_c$. The spline function progressively turns on the atom pair potentials between the outer radius $R_c$ and an inner radius $R_s$. We use a spline function with continuous first and second derivatives:

$$g(r) = \begin{cases} 1 & : \; r < R_s \\ -6x^5 + 15x^4 - 10x^3 + 1 & : R_s \leq r \leq R_c, x = \dfrac{r - R_s}{R_c - R_s} \\ 0 & : \; r > R_c \end{cases} \qquad \text{(Eq. 5.3.1.h)}$$

To complete the instructions for the calculation of $E_{disp}$, we need to derive the correction term $K / V_{cell}$. We first consider the interaction of an atom A in the asymmetric unit with the symmetry copies of an atom B. The average density of these symmetry copies is $\rho_B = m_B/V_{cell}$. Assuming a continuous distribution of the symmetry copies of B, one can calculate the missing interaction energy due to the use of a spline function (and a cut-off radius):

$$E_{cor,A,B} = -\frac{1}{2}\int_{R_s}^{\infty} f_{A,B}(r)\frac{C_{6,A,B}}{r^6}(1-g(r))4\pi r^2\rho_B dr \approx -\frac{2\pi m_B C_{6,A,B}}{V_{cell}}\int_{R_s}^{\infty}\frac{1-g(r)}{r^4}dr$$

$$\text{(Eq. 5.3.1.i)}$$

For the second (approximate) equality we have exploited the fact that $f_{A,B}$ is practically equal to one for large interatomic distances. The factor ½ reflects the fact that only half of the interaction energy is attributed to atom A to avoid double counting. To obtain the total correction energy per unit cell $E_{cor}$, we take into account that A has $m_A$ symmetry copies and let A and B run over all atoms in the asymmetric unit:

$$E_{cor} = \sum_A \sum_B m_A E_{cor,A,B} = \frac{K}{V_{cell}}$$
(Eq. 5.3.1.j)

$$K = -\int_{R_S}^{\infty} \frac{1-g(r)}{r^4} dr \sum_A \sum_B \frac{2\pi m_A m_B C_{6,A,B}}{V_{cell}}$$
(Eq. 5.3.1.k)

Using the spline function g(r) from (Eq. 5.3.1.h) and standard integration rules, the integral in (Eq. 5.3.1.k) can be determined analytically. Appropriate values for the inner spline radius and the cut-off radius are $R_s$=15Å and $R_c$=18Å.

### 5.3.2 Derivatives

The calculation of the first derivatives of $E_{disp}$ from equation (Eq. 5.3.1.a) with respect to the cell parameters $c_i$ and the atomic positions $\bar{v}_i$ is fairly straight forward:

$$\frac{\partial E_{disp}}{\partial c_i} = -\frac{K}{V_{cell}^2} \frac{\partial V_{cell}}{\partial c_i} + \sum_A \sum_B \sum_{j=1}^{m_B} \sum_{\substack{h,k,l \\ 0<|\Delta\vec{r}|\leq R_c}} \frac{1}{2} m_A \frac{\partial E_{A,B}}{\partial r}\left(|\Delta\vec{r}|\right)\frac{1}{|\Delta\vec{r}|}\Delta\vec{r}^T \frac{\partial \Delta\vec{r}}{\partial c_i}$$

(Eq. 5.3.2.a)

$$\frac{\partial E_{disp}}{\partial \vec{v}_i} = \sum_A \sum_B \sum_{j=1}^{m_B} \sum_{\substack{h,k,l \\ 0<|\Delta\vec{r}|\leq R_c}} \frac{1}{2} m_A \frac{\partial E_{A,B}}{\partial r}\left(|\Delta\vec{r}|\right)\frac{1}{|\Delta\vec{r}|}\left(\frac{\partial \Delta\vec{r}}{\partial \vec{v}_i}\right)^T \Delta\vec{r}$$
(Eq. 5.3.2.b)

In the second equation, $\partial E_{disp}/\partial \vec{v}_i$ is a column vector and $\partial \Delta r / \partial \vec{v}_i$ is a 3x3 matrix (see below). The derivative $\partial E_{A,B}/\partial \vec{r}$ is easily determined analytically using (Eq. 5.3.1.f) - (Eq. 5.3.1.h) and standard derivation rules. The cell volume $V_{cell}$ is related to the transformation matrix $\bar{L}$ via the following equation:

$$V_{cell} = \det(\bar{L}) = L_{xx}L_{yy}L_{zz} + L_{yx}L_{zy}L_{xz} + L_{zx}L_{xy}L_{yz} - L_{xz}L_{yy}L_{zx} - L_{yz}L_{zy}L_{xx} - L_{zz}L_{xy}L_{yx}$$
(Eq. 5.3.2.c)

Taking into account that $\partial \bar{L}/\partial c_i$ is known (see section 5.1), $\partial V_{cell}/\partial c_i$ in (Eq. 5.3.2.a) can be obtained from (Eq. 5.3.2.c) using standard derivation rules. The derivatives of $\Delta\vec{r}$ with respect to $c_i$ and $\vec{v}_i$ are given by the following equations:

$$\frac{\partial \Delta \vec{r}}{\partial c_i} = \frac{\partial \overline{L}}{\partial c_i}\left( \vec{x}_{fract,A} - \vec{x}_{fract,B,i} - \begin{pmatrix} h \\ k \\ l \end{pmatrix} \right) \qquad \text{(Eq. 5.3.2.d)}$$

$$\vec{x}_{fract,A} = \overline{S}_{A,0}\,(\overline{W}_A \vec{v}_A + w_A) + \vec{s}_{A,0} \qquad \text{(Eq. 5.3.2.de}$$

$$\vec{x}_{fract,B,j} = \overline{S}_{B,j}\,(\overline{W}_B \vec{v}_B + w_B) + \vec{s}_{B,j} \qquad \text{(Eq. 5.3.2.f)}$$

$$\frac{\partial \Delta \vec{r}}{\partial \vec{v}_i} = \overline{LS}_{A,0}\overline{W}_A\delta_{A,i}\overline{LS}_{B,j}\overline{W}_B\delta_{B,i} \qquad \text{(Eq. 5.3.2.g)}$$

In the last equation, $\delta_{a,b}$ is equal to one if a=b and 0 otherwise.

## 5.4 'Natural coordinates' for the lattice energy minimization of molecular crystals

In this section we present a coordinate system (called the natural coordinate system) that is more appropriate for lattice energy minimization than the coordinate system used in the previous sections (called the fractional coordinate system). Using the new coordinate system, energy minimization can be carried out using standard minimization routines such as the conjugate gradient technique [Press et al 2002]. The conjugate gradient technique requires the knowledge of the energy and its first derivatives. In the previous two sections, we have already shown how the energy and its first derivatives can be calculated in the fractional coordinate system. In this section, we will explain how natural coordinates can be converted to fractional coordinates and how energy derivatives obtained in fractional coordinates can be converted to energy derivatives with respect to natural coordinates.

### 5.4.1 Example - why the coordinate system is important

The choice of the coordinate system is crucial for the efficiency of the energy minimization process. To illustrate this point, let us consider the energy minimization of an isolated water molecule (see Fig. 4 which shows internal coordinates and Cartesian coordinates for water.). The point group for water is $C_{2v}$. Taking symmetry into account, we can describe the geometry of the water molecule by two parameters, for instance the H-O bond distance r and the H-O-H bond angle $\theta$ (internal coordinates). Alternatively, we may use the Cartesian coordinates x and y of one of the hydrogen atoms to describe the molecular geometry, keeping the oxygen atom fixed at the origin.
We define a potential energy surface in internal coordinates by the following expression:

$$E_{water}\,(r,\,\theta) = \frac{1}{2}\,4500\,\frac{kcal}{mol\text{\AA}^2}(r - 0.9572\text{\AA})^2 + \frac{1}{2}\,55\,\frac{kcal}{mol\,rad^2}(\theta\text{-}1.824\,rad)^2 \qquad \text{(Eq. 5.4.1.a)}$$

For illustration purposes we have deliberately chosen a value for the H-O stretch force constant that is 10 times higher than realistic values. The conversion to Cartesian coordinates is straight forward using:

$$r = \sqrt{x^2 + y^2} \qquad \text{(Eq. 5.4.1.b)}$$

$$\theta = 2\,\arcsin(x/r) \qquad \text{(Eq. 5.4.1.c)}$$

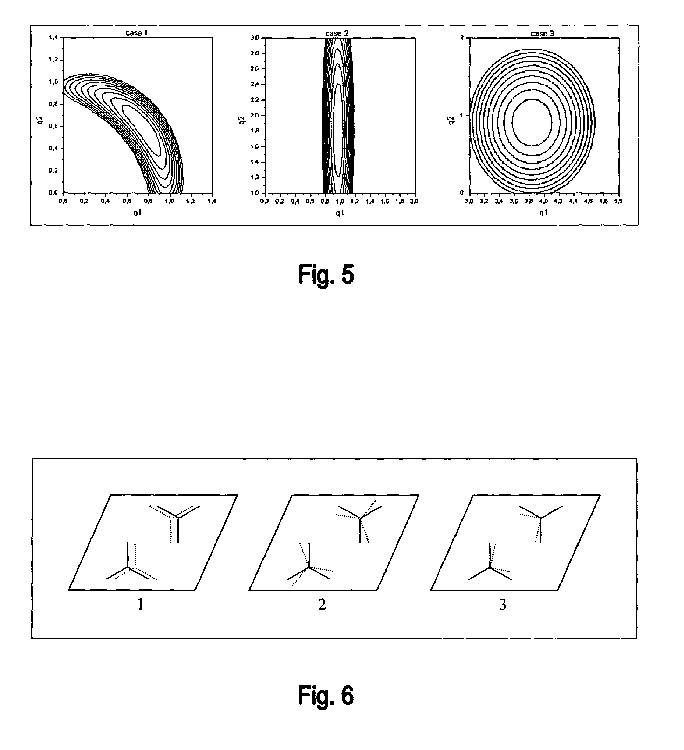

Fig. 5 shows contour plots of the potential energy surface for different definitions of the dimensionless parameters $q_1$ and $q_2$, wherein contour lines represent 10 kcal/mol intervals.
In Fig. 5, the potential energy surface is shown as a function of two dimensionless parameters $q_1$ and $q_2$ for

three different cases:

    1) Cartesian coordinates: $q_1=x/Å$, $q_2=y/Å$
    2) Internal coordinates: $q_1=r/Å$, $q_2=\theta/rad$
    3) Scaled internal coordinates: $q_1=4\ r/Å$ , $q_2=0.5\ \theta/rad$

In Cartesian coordinates (case 1) the potential energy surface is a curved valley. This kind of potential energy landscape is unfavourable for energy minimization. Most optimization algorithms proceed by a series of line searches. They first fall into the valley and than follow the valley to its lowest point. Since the valley is curved, this procedure may require a lot of steps. In internal coordinates (case 2), the potential energy surface is usually much more harmonic than in Cartesian coordinates. In our particular case, it is perfectly harmonic by defmition. In a harmonic potential well, the conjugate gradient algorithm finds the minimum in about n steps, where n is the number of variables. Using internal coordinates, the convergence is usually much faster than using Cartesian coordinates. In case 2, the potential energy well is harmonic, but the energy increases much faster in one direction than in the other. In extreme cases, this type of situation can lead to convergence problems. Using properly scaled internal coordinates (case 3), one obtains a more isotropic potential that is more favourable for energy minimization.

### 5.4.2 Natural coordinates for the lattice energy minimization of molecular crystals

In molecular crystals, atomic displacements of similar size can result in very different potential energy changes. On the one hand, structural changes the strongly affect bond lengths and bond angles are usually accompanied by important potential energy changes. On the other hand, there are concerted atomic motions that keep the bond lengths and bond angles constant while modifying intramolecular torsion angles and/or the molecular arrangement in the unit cell. Such concerted displacements are called weak modes as they result in large atomic displacements at small energetic cost. In Cartesian coordinates, weak modes often correspond to complex, curvilinear coordinate changes. In analogy to the water example discussed above, it is important for efficient lattice energy minimization to choose a coordinate system in which weak modes correspond to more or less straight lines.

In molecular crystals, intramolecular interactions are usually significantly stronger than intermolecular interactions. Therefore, weak modes and strong modes can be decoupled to a great extend by the use of separate coordinates for the description of whole molecule translations and rotations on the one hand and for the characterization of the molecular geometry on the other hand (see Fig. 6). Fig. 6 shows degrees of freedom describing atomic displacements in the unit cell: 1) Whole molecule translations, 2) whole molecule rotations, 3) intramolecular coordinate changes, the two molecules in the unit cell being symmetry related. To describe whole molecule translations, we use the position of the molecular centre (average over all atomic positions) in fractional coordinates. The orientation of the molecule is defined in terms of three successive rotations around mutually perpendicular axes. For the description of the intramolecular degrees of freedom we use delocalised internal coordinates which have shown to be an excellent choice for the structure optimization of isolated molecules [Baker 1997; Baker et al 1996].

So far, we have dealt with atomic displacements in a fixed unit cell. We now turn our attention to unit cell changes. Lattice deformations belong to the weakest modes in molecular crystals and it is important to choose a coordinate system in which the degrees of freedom relating to the unit cell and those relating to the atomic positions are more or less decoupled. The degree of coupling is strongly linked to the atomic displacements that are induced by unit cell changes. To illustrate this point, it is helpful to consider the case of fractional atomic coordinates as an example for a fairly inefficient choice of coordinates. If fractional coordinates are used to define the atomic positions in the unit cell, these fractional coordinates are usually held constant when the unit cell changes. As a consequence, unit cell changes, and in particular volume changes, are accompanied by strong changes of the covalent bond lengths resulting in strong changes of the net forces acting on the atoms. Cell parameters and atomic coordinates are thus strongly coupled.

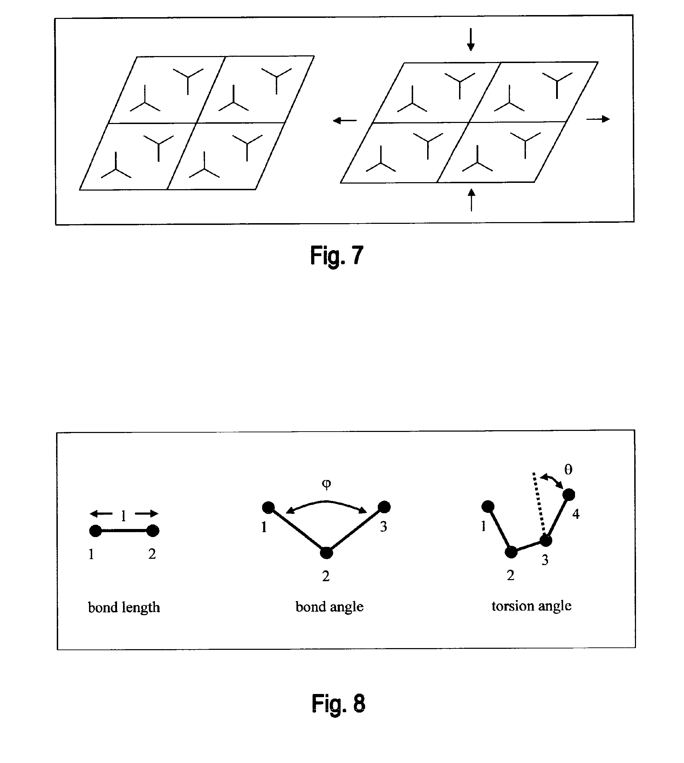

In this work, we describe unit cell changes in terms of an anisotropic expansion/compression of a starting cell along three mutually perpendicular axes (see next subsection and Fig. 7). Fig. 7 shows the molecular centres following the deformation of the lattice, while the molecular geometry and the molecular orientation remain unchanged. The molecular centres follow the expansion/compression (i.e. the fractional coordinates of the molecular centres remain constant) but the molecular geometries and orientations remain the same (i.e. the Cartesian coordinates of the atoms in a molecule with respect to the molecular centre remain constant). Our choice for the connection between lattice changes and atomic coordinate changes guarantees that lattice deformations only weakly affect the forces that are acting on the atoms in the unit cell. Let us suppose that the atomic coordinates correspond to the lattice energy minimum for a given set of unit cell parameters. In such a case, the forces on all atoms, and as a consequence the net forces and net torques on all molecules, are zero. If the unit cell changes, forces and torques change only little. The resulting net force on each molecule is small, because the expansion/compression along a given direction changes the intermolecular distances on both sides of the molecule in the same way. The resulting net torque on each molecule is small, because the expansion/compression does not induce a rotation of the molecule with respect to its neighbours. Finally, the change of the intermolecular distances results in small additional forces on all atoms in the unit cell, but these forces are much smaller than the forces that would have resulted from a change of the intramolecular distances rather than the intermolecular distances.

In the following four subsections, we present a more mathematical description of the natural coordinate system and its relationship with the fractional coordinate system.

### 5.4.3 Lattice changes

We describe lattice changes in terms of a transformation $\bar{M}$ from initial lattice vectors $\vec{a}_0$, $\vec{b}_0$ and $\vec{c}_0$ to new lattice vectors $\vec{a}$, $\vec{b}$ and $\vec{c}$ :

$$\vec{a} = \bar{M}\vec{a}_0 \ , \ \vec{b} = \bar{M}\vec{b}_0 \ , \ \vec{c} = \bar{M}\vec{c}_0 \qquad \text{(Eq. 5.4.3.a)}$$

$$\bar{L} = \bar{M}\bar{L}_0 \qquad \text{(Eq. 5.4.3.b)}$$

The vectors $\vec{a}_0$, $\vec{b}_0$ and $\vec{c}_0$ are oriented with respect to the external Cartesian coordinate system as described in section 5.1. The lattice vectors $\vec{a}_0$, $\vec{b}_0$ and $\vec{c}_0$ are the columns of the transformation matrix $\bar{L}_0$ from fractional atomic coordinates to Cartesian atomic coordinates.

The matrix $\bar{M}$ can always be decomposed into the product of an orthonormal matrix and a symmetric matrix (see (Eq. 5.5.1.d)- (Eq. 5.5.1.f)). The symmetric matrix corresponds to an anisotropic expansion/compression along three mutually perpendicular axes. The orthonormal matrix corresponds to a rotation of the crystal lattice. Since a rotation does not change the lattice parameters, we can drop the rotation without loss of generality and impose that the transformation matrix $\bar{M}$ is symmetric.

The new unit cell parameters a, b, c, $\alpha$, $\beta$, and $\gamma$ can be obtained from the new lattice vectors $\vec{a}$, $\vec{b}$ and $\vec{c}$ using the following equations:

$$a = \sqrt{\vec{a}^{T}\vec{a}} \qquad \text{(Eq. 5.4.3.c)}$$

$$b = \sqrt{\vec{b}^{T}\vec{b}} \qquad \text{(Eq. 5.4.3.d)}$$

$$c = \sqrt{\vec{c}^{T}\vec{c}} \qquad \text{(Eq. 5.4.3.e)}$$

$$\alpha = \frac{180°}{\pi}\arccos\left(\frac{\vec{b}^{T}\vec{c}}{bc}\right) \qquad \text{(Eq. 5.4.3.f)}$$

$$\beta = \frac{180°}{\pi}\arccos\left(\frac{\vec{a}^{T}\vec{c}}{ac}\right) \qquad \text{(Eq. 5.4.3.g)}$$

$$\gamma = \frac{180°}{\pi}\arccos\left(\frac{\vec{a}^{T}\vec{b}}{ab}\right) \qquad \text{(Eq. 5.4.3.h)}$$

The unit cell parameters can be converted to the independent, variable cell parameters $c_i$ by means of Tab. 5.1. a.

In all crystal systems apart from the triclinic one, the symmetric transformation matrix $\overline{M}$ must obey additional symmetry constraints. In the orthorhombic case, for instance, all of diagonal matrix elements must be zero because the lattice angles are fixed to 90°. We therefore decompose the transformation matrix $\overline{M}$ into a sum over independent, symmetry allowed transformations:

$$\overline{M} = \overline{I} + \sum_{i=1}^{nc} \lambda_i \kappa_\lambda \widetilde{M}_i \qquad \text{(Eq. 5.4.3.i)}$$

In the above equation, $\overline{I}$ is the identity matrix, $\kappa_\lambda$ is a scale factors and the matrices $\widetilde{M}_i$ are defined in Tab. 5.4.3.a for the various crystal systems. The parameters $\lambda_i$ are the coordinates that are used in the natural coordinate system to describe lattice changes. There are as many parameters $\lambda_i$ in the natural coordinate system as there are parameters $c_i$ in the fractional coordinate system.

Tab. 5.4.3.a: Components of the transformation matrix $\overline{M}$ used to describe lattice changes. In the trigonal case, $\vec{e}_1$, $\vec{e}_2$ and $\vec{e}_3$ are mutually perpendicular unit vectors with $\vec{e}_1$ being parallel to $\vec{a}_0 + \vec{b}_0 + \vec{c}_0$.

| Crystal system | nc | $\widetilde{M}_1$ | $\widetilde{M}_2$ | $\widetilde{M}_3$ | $\widetilde{M}_4$ | $\widetilde{M}_5$ | $\widetilde{M}_6$ |
|---|---|---|---|---|---|---|---|
| triclinic | 6 | $\begin{pmatrix} 1 & 0 & 0 \\ 0 & 0 & 0 \\ 0 & 0 & 0 \end{pmatrix}$ | $\begin{pmatrix} 0 & 0 & 0 \\ 0 & 1 & 0 \\ 0 & 0 & 0 \end{pmatrix}$ | $\begin{pmatrix} 0 & 0 & 0 \\ 0 & 0 & 0 \\ 0 & 0 & 1 \end{pmatrix}$ | $\begin{pmatrix} 0 & 1 & 0 \\ 1 & 0 & 0 \\ 0 & 0 & 0 \end{pmatrix}$ | $\begin{pmatrix} 0 & 0 & 1 \\ 0 & 0 & 0 \\ 1 & 0 & 0 \end{pmatrix}$ | $\begin{pmatrix} 0 & 0 & 0 \\ 0 & 0 & 1 \\ 0 & 1 & 0 \end{pmatrix}$ |
| monoclinic | 4 | $\begin{pmatrix} 1 & 0 & 0 \\ 0 & 0 & 0 \\ 0 & 0 & 0 \end{pmatrix}$ | $\begin{pmatrix} 0 & 0 & 0 \\ 0 & 1 & 0 \\ 0 & 0 & 0 \end{pmatrix}$ | $\begin{pmatrix} 0 & 0 & 0 \\ 0 & 0 & 0 \\ 0 & 0 & 1 \end{pmatrix}$ | $\begin{pmatrix} 0 & 0 & 1 \\ 0 & 0 & 0 \\ 1 & 0 & 0 \end{pmatrix}$ | - | - |
| orthorhombic | 3 | $\begin{pmatrix} 1 & 0 & 0 \\ 0 & 0 & 0 \\ 0 & 0 & 0 \end{pmatrix}$ | $\begin{pmatrix} 0 & 0 & 0 \\ 0 & 1 & 0 \\ 0 & 0 & 0 \end{pmatrix}$ | $\begin{pmatrix} 0 & 0 & 0 \\ 0 & 0 & 0 \\ 0 & 0 & 1 \end{pmatrix}$ | - | - | - |
| tetragonal | 2 | $\begin{pmatrix} 1 & 0 & 0 \\ 0 & 1 & 0 \\ 0 & 0 & 0 \end{pmatrix}$ | $\begin{pmatrix} 0 & 0 & 0 \\ 0 & 0 & 0 \\ 0 & 0 & 1 \end{pmatrix}$ | - | - | - | - |
| hexagonal | 2 | $\begin{pmatrix} 1 & 0 & 0 \\ 0 & 1 & 0 \\ 0 & 0 & 0 \end{pmatrix}$ | $\begin{pmatrix} 0 & 0 & 0 \\ 0 & 0 & 0 \\ 0 & 0 & 1 \end{pmatrix}$ | - | - | - | - |
| trigonal | 2 | $\vec{e}_1 \vec{e}_1^T$ | $\vec{e}_2 \vec{e}_2^T + \vec{e}_3 \vec{e}_3^T$ | - | - | - | - |
| cubic | 1 | $\begin{pmatrix} 1 & 0 & 0 \\ 0 & 1 & 0 \\ 0 & 0 & 1 \end{pmatrix}$ | - | - | - | - | - |

In subsection 5.4.1, we have seen that it is important to scale all coordinates in such a way the size of the potential energy well is roughly the same in all directions. In this work, the scale factor $\kappa_\lambda$ is set to 0.02.

**5.4.4 Whole molecule translations and rotations**

Crystal structures are usually defined in terms of lattice parameters and fractional atomic coordinates. Molecules are not specified at this basic level. The number of independent molecules as well as their composition and symmetry need to be derived from the fractional coordinates and the crystal symmetry. In this subsection, we first define a molecule in terms of its degrees of freedom and than discuss how the molecular composition, the molecular coordinate system and the molecular symmetry operations can be derived from the crystal symmetry and the fractional atomic coordinates.

For each molecule $\iota$, there is a set of $\eta_1$ symmetry independent atoms that constitute the molecular asymmetric unit. The position of each atom is specified in terms of a vector $\vec{\vartheta}_i$ with respect to a molecular Cartesian coordinate system. Each atom i in the asymmetric unit has $\mu_i$ symmetry copies at positions $\vec{\tilde{\vartheta}}_{i,j}$ with:

$$\vec{\tilde{\vartheta}}_{i,j} = \overline{\Gamma}_{i,j}\vec{\vartheta}_i \qquad \text{(Eq. 5.4.4.a)}$$

Here $\overline{\Gamma}_{I,j}$ is a 3x3 matrix and $\overline{\Gamma}_{i,0}$ is the identity matrix, i.e. $\vec{\tilde{\vartheta}}_{i,o} = \vec{\vartheta}_i$. In the molecular coordinate system, the geometrical centre of the molecule coincides with the origin:

$$\sum_{i=1}^{\eta_I}\sum_{j=1}^{\mu_i} \overline{\Gamma}_{i,j}\vec{\vartheta}_i = 0 \qquad \text{(Eq. 5.4.4.b)}$$

Some of the atoms in the asymmetric unit may lie on a symmetry element such as a rotation axis or a mirror plane. To consider only symmetry allowed atomic displacements, we decompose the Cartesian positions as follows:

$$\vec{\vartheta}_i = \vec{\vartheta}_{i,0} + \sum_{j=0}^{\sigma_i} \zeta_{i,j}\vec{\upsilon}_{i,j} \qquad \text{(Eq. 5.4.4.c)}$$

Here $\vec{\vartheta}_{i,0}$ is a reference positions. The $\sigma_i$ orthonormal vectors $\upsilon_{i,j}$ correspond to the symmetry allowed displacement directions and the dimensionless parameters $\zeta_{i,j}$ determine the atomic position. All vectors in (Eq. 5.4.4.c) are measured in Å.

Whole molecule rotations are defined in terms of np successive rotations $\overline{R}_i\,(\rho_i)$ around some or all of the axes of the molecular coordinate system. In addition, a rotation $\overline{R}_0$ needs to be applied to transform the atomic coordinates from the molecular coordinate system to the external Cartesian coordinate system. The overall rotation $\overline{R}$ is given by:

$$\overline{R} = \overline{R}_0 \prod_{i=1}^{n\rho} \overline{R}_i(\rho_i) \qquad \text{(Eq. 5.4.4.d)}$$

Due to symmetry constraints, there may be less than three successive rotations. For a molecule with a mirror plane, for instance, only rotations around an axis perpendicular to the mirror plane are possible. There may be 0, 1 or 3 allowed rotations and we always choose the molecular coordinate system in such a way that the rotation axes coincide with axes of the molecular coordinate system. Depending on the rotation axis, the matrices $\overline{R}_i\,(\rho_i)$ can take different forms specified in Tab. 5.4.4.a. The parameters $\rho_i$ are the coordinates used in the natural coordinate system to describe whole molecule rotations. All rotation matrices contain a scale factor $\kappa_\rho$. In this work we use $\kappa_\rho = 0.03$.

Tab. 5.4.4.a: Matrices describing rotations around axes of the molecular coordinate system. $\kappa_\rho$ is a scale factor and $\rho_i$ is a rotational coordinate in the natural coordinate system.

| Axis | $\overline{R}_i(\rho_i)=$ |
|---|---|
| X | $\begin{pmatrix} 1 & 0 & 0 \\ 0 & \cos\kappa_\rho\rho_i & \sin\kappa_\rho\rho_i \\ 0 & -\sin\kappa_\rho\rho_i & \cos\kappa_\rho\rho_i \end{pmatrix}$ |
| Y | $\begin{pmatrix} \cos\kappa_\rho\rho_i & 0 & \sin\kappa_\rho\rho_i \\ 0 & 1 & 0 \\ -\sin\kappa_\rho\rho_i & 0 & \cos\kappa_\rho\rho_i \end{pmatrix}$ |
| Z | $\begin{pmatrix} \cos\kappa_\rho\rho_i & \sin\kappa_\rho\rho_i & 0 \\ -\sin\kappa_\rho\rho_i & \cos\kappa_\rho\rho_i & 0 \\ 0 & 0 & 1 \end{pmatrix}$ |

In order to define the position of the molecule in the unit cell, we specify the position of the centre of geometry in fractional coordinates. If the centre of geometry overlaps with a symmetry element of the crystal structure, there are less than 3 independent move directions for the molecule. In analogy to (Eq. 5.4.4.c), we write:

$$\vec{T} = \vec{T}_0 + \sum_{i=1}^{\omega} \tau_i \kappa_\tau \vec{\xi}_i \qquad \text{(Eq. 5.4.4.e)}$$

Here $\overline{T}_0$ is a reference position, $\omega$ specifies the number of symmetry allowed move directions and $\kappa_\tau$ is a scale factor set to 0.1. The parameters $\tau_i$ specify the molecular position in the natural coordinate system. The independent move directions $\vec{\xi}_i$ are defined in such a way that the vectors $\overline{L}_0\vec{\xi}_i$ are orthonormal. Using (Eq. 5.4.4.d) and (Eq. 5.4.4.e), the atomic positions in molecular Cartesian coordinates can be converted to fractional atomic coordinates:

$$\vec{x}_{fract,i} = \vec{T} + \overline{L}^{-1} \overline{R} \vec{\vartheta}_i \qquad \text{(Eq. 5.4.4.f)}$$

The atoms in the molecular asymmetric units are mapped onto the atoms in the asymmetric unit of the crystal on a one to one basis. Each atom i in the asymmetric unit of the crystal is related to an atom at(i) in a molecule mol(i), and each atom i in a molecule j is related to an atom cry(i,j) in the asymmetric unit of the crystal:

$$\text{cry(at(i),mol(i)) = i} \qquad \text{(Eq. 5.4.4.g)}$$

$$\text{at(cry(i,j)) = i} \qquad \text{(Eq. 5.4.4.h)}$$

$$\text{mol(cry(i,j)) = j} \qquad \text{(Eq. 5.4.4.i)}$$

As there can be more than one independent molecule, most scalars, vectors and matrices in (Eq. 5.4.4.a) - (Eq. 5.4.4.f) should carry an additional index to indicate the molecule they refer to. However, for the sake of simplicity we omit the second index whenever this does not lead to confusion.

In general, it is necessary to apply a symmetry operation to an atom i in the asymmetric unit of the crystal in order to obtain the corresponding atom in the molecular asymmetric unit:

$$\vec{x}_{fract,i} = \overline{\overline{S}}_i(\overline{W}_i \vec{v}_i + \vec{w}_i) + \vec{\tilde{s}}_i \qquad \text{(Eq. 5.4.4.j)}$$

Combining (Eq. 5.4.4.f) and (Eq. 5.4.4.j), we obtain the transformation from molecular Cartesian coordinates to the coordinate vectors $v_i$, that describe the atomic positions in the factional coordinate system.

$$\vec{v}_i = \overline{W}_i^{-1}\left(\overline{\overline{S}}_i^{-1}\left(\vec{T}_{mol(i)} + \overline{L}^{-1}\overline{R}_{mol(i)}\vec{\vartheta}_{at(i),mol(i)} - \vec{\tilde{s}}_i\right) - \vec{w}_i\right) \qquad \text{(Eq. 5.4.4.k)}$$

The above equation allows us to describe the atomic displacements in the unit cell in terms of whole molecule translations (via $\vec{T}_{mol(i)}$), whole molecule rotations (via $\overline{R}_{mol(i)}$) and changes of the molecular structure (via $\vec{\vartheta}_{at(i),mol(i)}$). The coordinate transformations involves vectors (such as $\vec{\tilde{s}}_i$, $\vec{T}_0$, $\vec{\xi}_i$, etc), matrices (such as $\overline{\tilde{s}}_i$, $\overline{R}_0$, etc) and mappings (such as mol(i), at(i), etc) that need to be derived from the crystal symmetry and the initial positions of the atoms in the asymmetric unit. The second half of this subsection deals with the determination of the above mentioned objects.

As a starting point, we suppose that we know the fractional coordinates $x_i$ of all atoms in the asymmetric unit, their multiplicity $m_i$ and the corresponding symmetry operations (see (Eq. S.l.g)). Using this information, we can generate the atomic positions of all atoms in a central unit cell and the surrounding unit cells. Our first task is to identify the molecules that the atoms in the asymmetry unit belong to. For our purposes, we define a molecule as a set of atoms where every two atoms are connected via a series of covalent bonds. Two atoms i and j are covalently bonded if their distance is smaller than a certain multiple of the sum of their covalent radii. We use a multiple of 1.3 with the covalent bond radii presented in Tab. 5.4.4.a.

Tab. 5.4.4.b:

| Covalent bond radii for some elements. | |
| --- | --- |
| Element | Covalent radius [Å] |
| H | 0.299 |
| B | 0.830 |
| C | 0.767 |
| N | 0.702 |
| O | 0.659 |
| F | 0.619 |
| Cl | 1.023 |
| S | 1.052 |

To attribute all atoms in the asymmetric unit to a molecule, we start of with the first atom and we find all atoms in the central unit cell or in adjacent cells that are connected to the atom at the position $\overline{S}_{1,1}\vec{x}_1 + \vec{s}_{1,1}$ via a series of covalent bonds. We have now identified the first molecule which contains symmetry copies of a certain number of atoms in the asymmetric unit and which therefore determines the positions of these atoms. If there are any atoms in the asymmetric unit that do not belong to the first molecule, we take one of these atoms and we determine the corresponding second molecule. The procedure is repeated as long as there are atoms in the asymmetric unit that have not yet been attributed to a molecule. At the end of this procedure, the lookup table mol(i) is fully defined for all atoms i in the asymmetric unit.

Each molecule needs to be further processed. For the sake of simplicity, we again omit the index related to the number of the molecule in our discussion. Let us suppose that the molecule consists of n atoms at positions $x'_j$. It is straight forward to calculate the centre of geometry $\vec{T}_0$ that serves as a reference position for molecular translations:

$$\vec{T}_0 = \frac{1}{n}\sum_{j=1}^{n}\vec{x}'_j \qquad\qquad (\text{Eq. 5.4.4.l})$$

Knowing the molecular centre, we can determine the symmetry allowed translations and rotations. For a given space group, there are $m_{gen}$ general symmetry operations that can be described in terms of a matrix $\bar{S}_i$, and a translation $\vec{s}_i$ (see (Eq. 5.1.g)). We first determine the subset of the $m_{self}$ symmetry operations $(\bar{S}'_i, \vec{s}'_i)$ that project the molecular centre $\vec{T}_0$ onto itself to within a translation by a lattice vector:

$$\vec{T}_0 = \bar{S}_i\vec{T}_0 + \vec{s}'_i + \vec{t}_i \text{ with } \vec{t}_i = \begin{pmatrix} h_i \\ k_i \\ l_i \end{pmatrix} \qquad\qquad (\text{Eq. 5.4.4.m})$$

Here $h_i$, $k_i$ and $l_i$ must be integer numbers. A molecular displacement $\xi$ is symmetry allowed if and only if:

$$\vec{\xi} = \bar{S}'_i\vec{\xi} \text{ for all i with } 1 \leq i \leq m_{self} \qquad\qquad (\text{Eq. 5.4.4.n})$$

It is thus possible to determine a complete set of symmetry allowed displacements $\vec{\xi}_i$ by solving the system of linear equations defined by (Eq. 5.4.4.n). We define the displacement vectors $\vec{\xi}_i$ such that the vectors $\bar{L}_0\vec{\xi}_i$ are orthonormal.

We now turn our attention to the determinations of the symmetry allowed whole molecule rotations. For the discussion we choose a Cartesian coordinate system with the origin at $\vec{T}_0$ and axes parallel to the axes of the external Cartesian coordinate system. Any atom at a general position $\vec{r}$ in this coordinate system has $m_{self}$ symmetry copies at the following positions:

$$\vec{r}_i = \bar{Z}_i\vec{r} \text{ with } \bar{Z}_i = \bar{L}_0\bar{S}'_i\bar{L}_0^{-1} \qquad\qquad (\text{Eq. 5.4.4.o})$$

One of the symmetry operations $\bar{Z}_i$ must be the identity operation. If we carry out a small rotation by an angle $\Delta\varphi$ around an axis through the origin defined by a unit vector $\vec{n}$, the change of the vector $r$ is given in the linear approximation by:

$$\Delta\vec{r} = \Delta\varphi\,\vec{n}\times\vec{r} \qquad\qquad (\text{Eq. 5.4.4.p})$$

The coordinate change of the symmetry copies can be obtained either by applying the symmetry operations to (Eq. 5.4.4.p) or by applying the rotation to the result of (Eq. 5.4.4.o). Since both approaches must yield the same result, one obtains a series of conditions for the vector $\vec{n}$ :

$$\bar{Z}_i\,(\vec{n}\times\vec{r}) = \vec{n}\times(\bar{Z}_i\vec{r}) \text{ for all } \vec{r} \text{ and } 1 \leq i \leq m_{self} \qquad\qquad (\text{Eq. 5.4.4.q})$$

It can be shown that above conditions are fulfilled if and only if:

$$\vec{n}^T\vec{p}_{i,j} = 0 \text{ for all } \vec{p}_{i,j} \text{ from Tab. 5.4.4.c and } 1 \leq i \leq m_{self} \qquad\qquad (\text{Eq. 5.4.4.r})$$

Tab. 5.4.4.c: Vectors $\vec{p}_{i,j}$ required in (Eq. 5.4.4.r), $\overline{Z}_{i,jk}$ is an element of the matrix $\overline{Z}_i$.

| j | $\vec{p}_{i,j}$ | j | $\vec{p}_{i,j}$ | j | $\vec{p}_{i,j}$ |
|---|---|---|---|---|---|
| 1 | $\begin{pmatrix} 0 \\ -Z_{i,zx} - Z_{i,xz} \\ Z_{i,yx} + Z_{i,xy} \end{pmatrix}$ | 4 | $\begin{pmatrix} Z_{i,xz} \\ -Z_{i,zy} \\ Z_{i,yy} - Z_{i,xx} \end{pmatrix}$ | 7 | $\begin{pmatrix} -Z_{i,xy} \\ Z_{i,xx} - Z_{i,zz} \\ Z_{i,yz} \end{pmatrix}$ |
| 2 | $\begin{pmatrix} Z_{i,zx} \\ -Z_{i,yz} \\ Z_{i,yy} - Z_{i,xx} \end{pmatrix}$ | 5 | $\begin{pmatrix} Z_{i,zy} + Z_{i,yz} \\ 0 \\ -Z_{i,xy} - Z_{i,yx} \end{pmatrix}$ | 8 | $\begin{pmatrix} Z_{i,zz} - Z_{i,yy} \\ Z_{i,yx} \\ -Z_{i,xz} \end{pmatrix}$ |
| 3 | $\begin{pmatrix} -Z_{i,yx} \\ Z_{i,xx} - Z_{i,zz} \\ Z_{i,zy} \end{pmatrix}$ | 6 | $\begin{pmatrix} Z_{i,zz} - Z_{i,yy} \\ Z_{i,xy} \\ -Z_{i,zx} \end{pmatrix}$ | 9 | $\begin{pmatrix} -Z_{i,yz} - Z_{i,zy} \\ Z_{i,xz} + Z_{i,zx} \\ 0 \end{pmatrix}$ |

The vectors $\vec{p}_{i,j}$ span a subspace of the three-dimensional coordinate space. The dimension of this subspace can be 0, 2 or 3. If the dimension is 3, there are no symmetry allowed rotations. If the dimension is 2, there is one symmetry allowed rotation axis $\vec{n}_i$. Finally, if the dimension is 0, all rotations are allowed. Knowing the vectors $\vec{p}_{i,j}$, the allowed rotation directions are easily calculated.

We now turn to the determination of the transformation matrix $\bar{R}_0$ from the molecular Cartesian coordinate system to the coordinate system with the origin at $\vec{T}_0$ and axes parallel to the axes of the external coordinate system (shifted external coordinate system). We always chose the matrix $\bar{R}_0$ such that the axes of the molecular coordinate system are parallel to the principal axes of the 'inertia tensor' calculated with all masses set to 1 for the initial molecular geometry. The molecular x-axis and z-axis correspond to the smallest and the largest 'moment of inertia', respectively. The principal axes and the principal moments reflect the shape at the molecule and it can be expected that there is some kind of correlation between these axes on the one hand and the directions of weakly/strongly hindered rotations on the other hand. It is therefore advisable to use the principal axes as rotation axes for whole molecule rotations.

We define the 'tensor of inertia' with respect to the shifted external coordinate system as follows:

$$\overline{\Theta} = \sum_{j=1}^{n} \left| \overline{L}_0(\vec{x}'_j - \overline{T}_0) \right|^2 \overline{I} - \overline{L}_0(\vec{x}'_j - \overline{T}_0)(\vec{x}'_j - \overline{T}_0)^T \overline{L}_0^T \qquad \text{(Eq. 5.4.4.s)}$$

$\bar{R}_0$ is

the orthonormal matrix that diagonalizes $\overline{\Theta}$, i.e. the eigenvectors of $\overline{\Theta}$ are the columns of $\bar{R}_0$:

$$\overline{\Theta}\bar{R}_0 = \bar{R}_0\Lambda \qquad \text{(Eq. 5.4.4.t)}$$

Here $\Lambda$ is a diagonal matrix with $\Lambda_{xx} \leq \Lambda_{yy} \leq \Lambda_{zz}$. Since we describe whole molecule rotations in terms of rotations around the axes of the molecular coordinate system, we always have to make sure that the allowed rotations axes coincide with the molecular axes. This is automatically the case if all or no whole molecule rotations are allowed, but the case of a single allowed rotation axis requires further discussion. In principle, the allowed rotation axis always corresponds to a principal axis due to symmetry considerations. However, it may happen accidentally that the 'tensor of inertia' has two or three degenerate (=identical) eigenvalues. In this case, standard diagonalization routines do not necessarily yield eigenvectors with one eigenvector parallel to the allowed rotation axis and the eigenvectors need to be redefined. If no eigenvector is parallel to the allowed rotation axis, we distinguish two cases:

- Allowed rotation axis $\vec{n}$ perpendicular to one of the eigenvectors $\vec{e}$. The new eigenvectors are $\vec{n}$, $\vec{e}$ and a vector perpendicular $\vec{n}$ and $\vec{e}$.
- Allowed rotation axis $\vec{n}$ perpendicular to none of the eigenvectors. The new eigenvectors are $\vec{n}$ and two additional vectors perpendicular to $\vec{n}$ and to each other.

Once the transformation matrix $\bar{R}_0$ is determined, it is straight forward to choose the rotation matrices $\bar{R}_i$. If there is a single allowed rotation, $\bar{R}_i$ must correspond to the allowed rotation axis in the molecular coordinate system. If all three rotations are allowed, we choose $\bar{R}_t = \bar{R}_x$, $\bar{R}_2 = \bar{R}_y$ and $\bar{R}_3 = \bar{R}_z$. In certain cases, for instance when dealing with linear rigid molecules, it can be appropriate to disable the rotation around the axis with the smallest 'moment of inertia'

We still need to specify the atoms in the molecular asymmetry unit, their mapping onto the atoms in the asymmetric unit of the crystal and their symmetry copies within the molecule. To obtain this information, we examine all atoms of the molecule one by one. Let us suppose that we are considering atom j of the molecule at the position $x'_j$ which is related to the m-th symmetry copy of atom i in the asymmetric unit of the crystal via:

$$\vec{x'}_j = \bar{S}_{i,m}(\bar{W}_i\vec{v}_i + \vec{w}_i) + \vec{s}_{i,m} + \vec{t}_{i,m,j} \qquad \text{(Eq. 5.4.4.u)}$$

We need to distinguish two cases:

**Case 1**: So far, we have not come across an atom within the molecule that is related to the atom i in the asymmetric unit of the crystal. We add the atom j to the atoms in the molecular asymmetric unit. The atom gets a new index j' which is equal to the current number of atoms in the asymmetric unit. We set cry(j',mol)=i and at(i)=j'. For the symmetry elements that relate atom j' in the molecular asymmetric unit to atom i in the asymmetric unit of the crystal we obtain:

$$\bar{\bar{S}}_i = \bar{S}_{i,m} \ , \ \vec{\bar{s}}_i = \vec{s}_{i,m} + \vec{t}_{i,m,j} \qquad \text{(Eq. 5.4.4.v)}$$

The position of atom j' in the molecular Cartesian coordinate system is:

$$\vec{v}_{j',0} = \bar{R}_0^{-1}\bar{L}_0(\vec{x'}_j - \vec{T}_0) \qquad \text{(Eq. 5.4.4.w)}$$

Symmetry allowed atomic move directions for atom j' can be obtained from the following equation:

$$\vec{v}'_{j',k} = \bar{R}_0^{-1}\bar{L}_0\bar{\bar{S}}_i\bar{W}_i\vec{e}_k \ , \ 1 \le k \le \sigma_{j'} \ , \ \vec{e}_k = \begin{pmatrix} 1 \\ 0 \\ 0 \end{pmatrix} \leftarrow k \qquad \text{(Eq. 5.4.4.x)}$$

Here only such vectors $\vec{e}_k$ are allowed that are compatible with the definition of the relevant (non-zero) components of the vectors $\vec{v}_i$ in section 5.1. The vectors $\vec{v}_{j',k}$ can be obtained from the vectors $\vec{v}'_{j,k}$ by orthonormalization.

Whenever we add a new atom j' to the molecular asymmetric unit, we set the number of symmetry copies $\mu$ of atom j' to 1. There is always at least one symmetry copy $\bar{\Gamma}_{j',1}$ which is equal to the identity matrix.

**Case 2**: We have already come across an atom $\tilde{j}$ that is related to the m'-th symmetry copy of atom i in the asymmetric unit of the crystal by (Eq. 5.4.4.u). The atom $\tilde{j}$ has become atom $\tilde{j}'$ in the molecular asymmetric unit. The atom j currently under consideration is a symmetry copy of atom $\tilde{j}'$. We thus increase the number of symmetry copies $\mu$ of $\tilde{j}'$ by one. The new symmetry operation is given by:

$$\overline{\Gamma}_{\widetilde{j}',\mu} = \overline{R}_0^{-1}\overline{L}_0\overline{S}_{i,m}\overline{S}_{i,m}^{-1}.\overline{L}_0^{-1}\overline{R}_0 \qquad\qquad \text{(Eq. 5.4.4.y)}$$

The procedure outline above is repeated until all atoms of the molecule have been assigned to an new atom in the molecular asymmetric unit (case 1) or to the symmetry copy of an existing atom in the molecular asymmetric unit (case 2).

**5.4.5 Delocalized internal coordinates**

In the previous section, we have defined the intramolecular degrees of freedom in terms of atomic displacement parameters $\zeta_{i,j}$ where the index i refers to the atom number in the molecular asymmetric unit and the index j refers to the $\sigma_i$ symmetry allowed Cartesian displacements of atom i. In the following, we will define the molecular conformation in terms of a symmetry adapted Cartesian coordinate vector $\vec{\zeta}$ that is composed of the individual parameters $\zeta_{i,j}$ :

$$\vec{\zeta} = (\zeta_{1,1},...,\ \zeta_{1,\sigma_i},\ \zeta_{2,1},...,\ \zeta_{2,\sigma_2},\ ...) \qquad\qquad \text{(Eq. 5.4.5.a)}$$

As we have seen in section 5.4.1, Cartesian coordinates are not the most efficient way to describe intramolecular degrees of freedom. In this subsection, we introduce internal delocalized coordinates $\vec{\theta} = (\theta_1,...,\theta_{n\theta})$ that are more appropriate for energy minimization. In our discussion, we adapt the approach outlined in Baker et al. 1996 to the case of a molecule on a general (no internal symmetry elements) or a special position (internal symmetry elements) in a crystalline environment.

In many computer programs, the so called Z-matrix approach is used in order to define the molecular geometry. The Z-matrix is nothing else but a non-redundant set of intramolecular coordinates such as bond lengths, bond angles and torsion angles which completely define the molecular geometry. For the energy minimization of complex molecules (covalently bonded rings in particular), the Z-matrix approach is often quite inefficient, since the arbitrary selection of internal coordinates used to describe the molecular geometry does not correspond to the best possible choice. Baker et al. describe the molecular geometry in terms of a non-redundant set of delocalized internal coordinates, where each delocalized coordinate is a linear combination of all localized internal coordinates (bond length, bond angles, etc.). The main merit of their approach is to provide a straight forward procedure for the construction of 'the best possible set' of internal delocalized coordinates for any molecule.

Following Baker et al, we consider a set of nq internal coordinates $\bar{q} = (q_1,..., q_{nq})^T$ . In general, we chose all intramolecular bond lengths, bond angles and torsion angles. Symmetry related internal coordinates are considered only once. Bond angles close to 180° require special attention. The determination of internal coordinates and their first derivatives as a function of the Cartesian atomic coordinates will be dealt with at the end of this section.

Small changes $\Delta q$ of the internal coordinates are related to small Cartesian displacements $\Delta\vec{\zeta}$ by means of the B matrix:

$$\Delta\vec{q} = \bar{B}\Delta\vec{\zeta} \qquad\qquad \text{(Eq. 5.4.5.b)}$$

$$\bar{B} = \begin{pmatrix} \partial q_1/\partial\zeta_1 & \cdots & \partial q_1/\partial\zeta_{n\zeta} \\ \vdots & \ddots & \vdots \\ \partial q_{nq}/\partial\zeta_1 & \cdots & \partial q_{nq}/\partial\zeta_{n\zeta} \end{pmatrix} \qquad\qquad \text{(Eq. 5.4.5.c)}$$

In general, there are fare more internal coordinates $q_i$ than allowed Cartesian move directions $\zeta_i$. As we want to describe the molecular geometry in terms of internal coordinates, it is important to remove this redundancy. An elegant way to remove redundancies is to diagonalize the nq x nq matrix $\bar{G} = \bar{B}_0\bar{B}_0{}^T$, where $\bar{B}_0$ is the B matrix obtained for the initial molecular geometry $\vec{q}_0$. Non-redundant directions are readily identified as the $n\theta$ mutually perpendicular eigenvectors $\vec{\psi}_i$ of $\bar{G}$ with eigenvalues greater than zero. The normalization of the eigenvector $\vec{\psi}_i$ is discussed later. By means of the eigenvectors $\vec{\psi}_i$, one can define $n\theta$ non redundant coordinates $\theta_i$ :

$$\theta_i = \vec{\psi}_i^T (\vec{q} - \vec{q}_0) \qquad \text{(Eq. 5.4.5.d)}$$

As the coordinates $\theta_i$ are linear combinations of many primitive internal coordinates, they are called delocalized internal coordinates. These are the coordinates used in the natural coordinate system to describe the molecular geometry.

Small Cartesian coordinate changes $\Delta\vec{\zeta}$ can be easily converted to changes of the delocalized atomic coordinates $\Delta\vec{\theta} = (\Delta\theta_1,..., \Delta\theta_{n\theta})$:

$$\Delta\vec{\theta} = \overline{\overline{B}}\Delta\vec{\zeta} \qquad \text{(Eq. 5.4.5.e)}$$

$$\overline{\overline{B}} = \overline{U}^T * \overline{B} \qquad \text{(Eq. 5.4.5.f)}$$

$$\overline{U} = (\vec{\psi}_0,...,\vec{\psi}_{n\theta}) \qquad \text{(Eq. 5.4.5.g)}$$

Here the vectors $\vec{\psi}_i$ form the columns of the matrix $\overline{U}$. It is important to note that there are, in general, more symmetry allowed Cartesian displacements than delocalized internal coordinates. This is due to the fact that whole molecule translations and rotations change the Cartesian coordinates, but not the internal coordinates. Consequently, $\overline{\overline{B}}$ is not a square matrix and cannot be directly inverted. However, it is possible to construct an 'inverse B matrix' in the following way:

$$\overline{\overline{B}}^{-1} = \overline{\overline{B}}^T (\overline{\overline{B}}\,\overline{\overline{B}}^T)^{-1} \qquad \text{(Eq. 5.4.5.h)}$$

$$\Delta\vec{\zeta} = \overline{\overline{B}}^{-1}\Delta\vec{\theta} \qquad \text{(Eq. 5.4.5.i)}$$

The 'inverse B matrix' defined in (Eq. 5.4.5.h) yields Cartesian displacements without any translational or rotational component.

The transformation from delocalized internal coordinates to Cartesian coordinates is not linear and (Eq. 5.4.5.i) only holds for small coordinate changes. For large coordinate changes, we use an iterative procedure to transform delocalised internal coordinates to Cartesian coordinates. Let us suppose that the Cartesian coordinates $\bar{\zeta}$ correspond to the delocalized internal coordinates $\bar{\theta}$ and that we would like to know the Cartesian coordinates $\bar{\zeta}'$ that correspond to new delocalized internal coordinates $\vec{\theta}'$. We use the Cartesian coordinates $\vec{\zeta}$ as a starting point and set $\vec{\zeta}(0) = \vec{\zeta}$. Here zero indicates the start of the iteration. Given the Cartesian coordinates $\vec{\zeta}(k)$, one can calculate the corresponding delocalized internal coordinates $\vec{\theta}(k)$ and the corresponding inverse B-matrix $\overline{\overline{B}}^{-1}(k)$. It is then possible to exploit the linear relationship for small coordinate changes to obtain a better estimate for the Cartesian coordinates that match $\theta'$:

$$\vec{\zeta}(k+1) = \vec{\zeta}(k) + \overline{\overline{B}}^{-1}(k)\left[\vec{\theta}' - \vec{\theta}(k)\right] \qquad \text{(Eq. 5.4.5.j)}$$

The iterative procedure must be repeated until the Cartesian coordinate changes become negligible small. The iteration normally converges very rapidly and convergence to within $10^{-10}$ Å is typically obtained after 3-4 cycles. The speed of convergence obviously depends on the quality of the starting point for the iterative procedure. For energy minimizations, we always use the structure with the currently lowest energy as a starting point.

The iterative procedure outlined above yields Cartesian coordinates $\vec{\zeta}'$ that match the delocalized internal coordinates $\vec{\theta}'$, but the Cartesian coordinates $\vec{\zeta}'$ are not uniquely defined. If whole molecule translations and rotations are not forbidden by symmetry constraints, the repeated use of the iterative procedure results in ill-defined whole molecule translations and rotations in the molecular reference system. A small part of these ill-defined whole molecule displacements stems from the accumulation of rounding errors. The main effect, however, is related to the fact that the overall rotation of the molecule depends on the pathway followed in $\vec{\theta}$ space. If one changes the geometry from $\vec{\theta}$ to $\vec{\theta}'$, from $\vec{\theta}'$ to $\vec{\theta}''$ and from $\vec{\theta}''$ back to $\vec{\theta}$, an overall rotation of the molecule is induced that depends on the conformations $\vec{\theta}'$ and $\vec{\theta}''$.

When delocalized internal coordinates are used for the structure optimization of isolated molecules, the occurrence of ill-defined whole molecule displacements does not need to be taken into account, as such displacements do not result in potential energy changes. It is probably for this reason that the problem was not mentioned in the work of Baker et al. In the case of crystal structure optimization however, such ill-defined whole molecule displacements in the molecular reference system can not be tolerated, as they affect the interatomic distances and thus the potential energy. We solve the problem by introducing a additional whole molecule translations and rotations which depend on the Cartesian displacements $\vec{\zeta}$ and insure that the molecule always adopts a well-defined position and orientation.

To start with, we replace (Eq. 5.4.4.c) by the following expression:

$$\vec{\vartheta}_i' = \vec{\vartheta}_{i,0} + \sum_{j=0}^{\sigma_i} \zeta_{i,j} \vec{\upsilon}_{i,j} \qquad\qquad \text{(Eq. 5.4.5.k)}$$

We then transform the ill-defined Cartesian coordinates $\vec{\vartheta}_i'$ to well-defined Cartesian coordinates $\vec{\vartheta}_i$:

$$\vec{\vartheta}_i = \overline{R}_{corr}(\vec{\vartheta}_i - \vec{\vartheta}_{corr}') \qquad\qquad \text{(Eq. 5.4.5.l)}$$

$$\vec{\vartheta}_{corr}' = \frac{1}{n} \sum_{i=1}^{\eta_t} \sum_{j=1}^{\mu_i} \overline{\Gamma}_{i,j} \vec{\vartheta}_i' \qquad\qquad \text{(Eq. 5.4.5.m)}$$

$$\overline{R}_{corr} = \prod_{i=1}^{n\rho} \overline{R}_{i,corr}(\varphi_i) \qquad\qquad \text{(Eq. 5.4.5.n)}$$

$$\vec{n}_k^T \left( \sum_{i=1}^{\eta_t} \sum_{j=1}^{\mu_i} \overline{\Gamma}_{i,j} \vec{\vartheta}_{i,0} \times \overline{\Gamma}_{i,j} \overline{R}_{corr}(\vec{\vartheta}_i' - \vec{\vartheta}_{corr}') \right) = 0 \quad \text{for all rotation directions} \qquad \text{(Eq. 5.4.5.o)}$$

In (Eq. 5.4.5.m), n is the total number of atoms in the molecule, i.e. the sum over all multiplicities $\mu_i$. By subtraction $\vec{\vartheta}_{corr}'$ from all $\vec{\vartheta}_i'$, we make sure that the centre of the molecule always coincides with the origin of the molecular Cartesian coordinate system. The matrix $\overline{R}_{corr}$ rotates the molecule so that it adopts a well defined orientation. $\overline{R}_{corr}$ is the product of $n\rho$ matrices (see (Eq. 5.4.5.n)) that are identical to the rotation matrices in (Eq. 5.4.4.d), apart from the fact that the rotation angles $\rho_i$ have been replaced by rotation angles $\varphi_i$. (Eq. 5.4.5.o) uniquely defines the rotation matrix $\overline{R}_{corr}$, as there are as many equations as rotation angles $\varphi_i$. The vectors $\vec{n}_k$ are unit vectors parallel to the symmetry allowed rotation axes in the molecular reference frame.

The determination of the rotation angles $\varphi = (\varphi_1, ..., \varphi_{np})$ requires some additional explanations, as (Eq. 5.4.5.n), (Eq. 5.4.5.o) and Tab. 5.4.4.a define a complex set of non-linear equations. In general, we already know the Cartesian displacements $\vec{\zeta}$ and the rotation angles $\varphi$ that correspond to the delocalised internal coordinates $\theta$, and we would like to determine the vectors $\vec{\zeta}'$ and $\varphi'$ that correspond to new delocalized internal coordinates $\theta'$. The change or the rotation correction from $\varphi$ to $\varphi'$ is fairly small in most cases. We therefore use an iterative procedure consisting of a series of linear approximations to determine $\varphi$. To start with, we set $\vec{\varphi}(0) = \vec{\varphi}$. At each iteration, we first calculate $\overline{R}_{corr}(l)$ and $\partial \overline{R}_{corr}/\partial(\varphi_i(l))$ for the current rotation correction $\vec{\varphi}(l)$. We then calculate the new rotation correction according to the equations below:

$$\vec{\phi}(l+1) = \vec{\phi}(l) - \overline{M}^{-1}\vec{m} \qquad \text{(Eq. 5.4.5.p)}$$

$$\vec{m} = (m_l,...,m_{n\rho})^T \qquad \text{(Eq. 5.4.5.q)}$$

$$m_k = \vec{n}_k^T\left(\sum_{i=1}^{\eta_i}\sum_{j=1}^{\mu_i}\overline{\Gamma}_{i,j}\vec{\vartheta}_{i,0}\times\overline{\Gamma}_{i,j}\overline{R}_{corr}(\vec{\vartheta}_i'-\vec{\vartheta}_{corr}')\right) \qquad \text{(Eq. 5.4.5.r)}$$

$$\overline{M} = \begin{pmatrix} M_{1,1} & \cdots & M_{1,n\rho} \\ \vdots & \ddots & \vdots \\ M_{n\rho,1} & \cdots & M_{n\rho,n\rho} \end{pmatrix} \qquad \text{(Eq. 5.4.5.s)}$$

$$M_{k,h} = \vec{n}_k^T\left(\sum_{i=1}^{\eta_i}\sum_{j=1}^{\mu_i}\overline{\Gamma}_{i,j}\vec{\vartheta}_{i,0}\times\overline{\Gamma}_{i,j}\frac{\partial\overline{R}_{corr}}{\partial\varphi_h}(\vec{\vartheta}_i'-\vec{\vartheta}_{corr}')\right) \qquad \text{(Eq. 5.4.5.t)}$$

The iterative procedure must be repeated until the Cartesian coordinate changes induced by the rotation $\overline{R}_{corr}$ become negligible small (see (Eq. 5.4.5.1)). The iteration normally converges very rapidly and convergence to within $10^{-10}$ Å is typically obtained after 3-4 cycles.

In the first part of this section, we have provided an overview over the essential mathematical concepts required to define delocalised internal coordinates and to convert delocalised internal coordinates to Cartesian coordinates. We now add some important details that have been held back so far to simplify the above discussion.

We first turn our attention to the normalization of the eigenvectors $\vec{\psi}_i$ of the G matrix. Delocalised internal coordinates are defined with respect to a certain starting geometry given by the atomic positions $\vartheta_{i,0}$. For this starting geometry ($\zeta_0$, $\varphi_0$ and $\theta_0$ are all zero. A unit change $\Delta\theta_i =1$ with respect to the starting geometry leads to Cartesian displacements $\Delta\zeta_i$ that are given in the linear approximation by the following equation:

$$\Delta\vec{\zeta}_i = \frac{1}{\lambda_i(\vec{\psi}_i^T\vec{\psi}_i)}\overline{B}_0^T\vec{\psi}_i \qquad \text{(Eq. 5.4.5.u)}$$

Here $\lambda_i$ is the eigenvalue for the eigenvector $\psi_i$. The length of $\psi_i$ is thus inversely related to the Cartesian displacements induced by a unit change $\Delta\theta_i =1$. We normalize $\psi_i$, with respect to the norm of $\Delta\zeta_i$:

$$\left|\Delta\vec{\zeta}_i\right| = \left|\frac{1}{\lambda_i(\vec{\psi}_i^T\vec{\psi}_i)}\overline{B}_0^T\vec{\psi}_i\right| = \kappa_\theta \qquad \text{(Eq. 5.4.5.v)}$$

We have seen in section 5.4.1 that coordinates used for energy minimization should be scaled in such a way that a unit displacement away from the energy minimum results in roughly the same energy change for all coordinates. Performing calculations for several molecular crystals, we have found that a value of $\kappa_\theta = 0.033$ is roughly compatible with the values $\kappa_\rho=0.03$, $\kappa_\lambda=0.02$ and $\kappa_\tau=0.1$ used to scale the other coordinate types. A more thorough parameterization of the scale factors is planned.

The next item we need to discuss is the diagonalization of the G matrix. In most cases, there are significantly more internal coordinate than Cartesian displacement directions. We follow [Andzelm et al 2001] and diagonalize the smaller matrix $\bar{F} = \bar{B}_0{}^T \bar{B}_0$ instead of the bigger matrix $\bar{G} = \bar{B}_0 \bar{B}_0{}^T$ :

$$\overline{FS} = \overline{S\Lambda} \qquad \text{(Eq. 5.4.5.w)}$$

Here $\bar{S}$ is the matrix of eigenvectors with non-zero eigenvalues and $\bar{\Lambda}$ is the corresponding diagonal matrix of eigenvalues. The matrix $\bar{U}$ that contains the eigenvectors of $\bar{G}$ is easily obtained in a second step:

$$\bar{U} = \bar{B}_0 \overline{S\Lambda}^{-1/2} \qquad \text{(Eq. 5.4.5.x)}$$

A central task when dealing with delocalized internal coordinates is the calculation of the B matrix. In most cases, we use a complete list of bond lengths, bond angles and torsion angles. Symmetry related internal coordinates are considered only once. The following equations relate the bond length 1 and the bond angle $\varphi$ (measured in rad) to the Cartesian coordinates of the corresponding atoms. The atom numbers are defined in Fig. 8. Fig. 8 illustrates the definition of a bond length, a bond angle and a torsion angle.

$$l = \sqrt{(\vec{x}_1 - \vec{x}_2)^T (\vec{x}_1 - \vec{x}_2)} \qquad \text{(Eq. 5.4.5.y)}$$

$$\varphi = \arccos\left( \frac{(\vec{x}_1 - \vec{x}_2)^T (\vec{x}_3 - \vec{x}_2)}{|\vec{x}_1 - \vec{x}_2||\vec{x}_3 - \vec{x}_2|} \right) \qquad \text{(Eq. 5.4.5.z)}$$

The mathematical definition of a torsion angle $\theta$ is a little bit more complicated. We first define two vectors $\vec{v}_1$ and $\vec{v}_2$ that are then used to define the torsion angle $\theta$. Positive torsion angles correspond to clockwise rotations when looking down the axis from atom 2 to atom 3.

$$\vec{v}_1 = (\vec{x}_1 - \vec{x}_2) - \frac{((\vec{x}_1 - \vec{x}_2)^T (\vec{x}_2 - \vec{x}_3))}{|\vec{x}_2 - \vec{x}_3|} \frac{(\vec{x}_2 - \vec{x}_3)}{|\vec{x}_2 - \vec{x}_3|} \qquad \text{(Eq. 5.4.5.a2)}$$

$$\vec{v}_2 = (\vec{x}_4 - \vec{x}_3) - \frac{((\vec{x}_4 - \vec{x}_3)^T (\vec{x}_2 - \vec{x}_3))}{|\vec{x}_2 - \vec{x}_3|} \frac{(\vec{x}_2 - \vec{x}_3)}{|\vec{x}_2 - \vec{x}_3|} \qquad \text{(Eq. 5.4.5.b2)}$$

$$\theta = \begin{cases} 2\pi n + \arccos \dfrac{\vec{v}_1^T \vec{v}_2}{|\vec{v}_1||\vec{v}_2|} & : \quad ((\vec{x}_1 - \vec{x}_2) \times (\vec{x}_3 - \vec{x}_2))^T (\vec{x}_4 - \vec{x}_3) \le 0 \\[3mm] 2\pi(n+1) - \arccos \dfrac{\vec{v}_1^T \vec{v}_2}{|\vec{v}_1||\vec{v}_2|} & : \quad ((\vec{x}_1 - \vec{x}_2) \times (\vec{x}_3 - \vec{x}_2))^T (\vec{x}_4 - \vec{x}_3) > 0 \end{cases} \qquad \text{(Eq. 5.4.5.c2)}$$

To avoid discontinuities at eclipsed atomic configurations, we attribute a counter n to each torsion angle that counts the number of full rotations and is increased/decreased by one each time the eclipsed atomic configuration is crossed.

To obtain the internal coordinates as a function of the Cartesian displacements $\vec{\zeta}$, the vectors $\vec{x}_i$, in (Eq. 5.4.5.y)- (Eq. 5.4.S.c2) need to be replaced by the appropriate vectors $\tilde{\vec{a}}_{i,j}$ which are related to the Cartesian displacements $\vec{\zeta}$ via (Eq. 5.4.4.a) and (Eq. 5.4.4.c). The derivatives of the internal coordinates with respect to the components of $\vec{\zeta}$ can be obtained by the application of standard derivation rules.

A particular situation arises when a bond angle approaches $\pi$. In this case, the first derivatives of the bond angle with respect to the Cartesian coordinates diverge, and some torsion angles may become ill defined. If the central atom has more than two neighbours - a situation frequently encountered when dealing with metallo-organic complexes or inorganic compounds - it is often possible to simply drop to ill-behaved internal coordinates, as the remaining internal coordinates are sufficient to characterize the molecular geometry completely. If the central atom has two neighbours, we also drop all ill-behaved internal coordinates. In addition, we define two reference directions $\vec{e}_1$ and $\vec{e}_2$ that are mutually perpendicular and roughly perpendicular to the axis that runs through the two neighbouring atoms. The two reference directions allow us to define two new internal coordinates $\tilde{\varphi}_1$ and $\tilde{\varphi}_2$ that describe the deviation of the central atom and its two neighbours from a straight line. Both internal coordinate are the sum of two 'bond angles':

$$\widetilde{\varphi}_i = \arccos \frac{\vec{e}_i^T (\vec{x}_1 - \vec{x}_2)}{|\vec{e}_i||\vec{x}_1 - \vec{x}_2|} + \arccos \frac{\vec{e}_i^T (\vec{x}_3 - \vec{x}_2)}{|\vec{e}_i||\vec{x}_3 - \vec{x}_2|} \qquad \text{(Eq. 5.4.5.d2)}$$

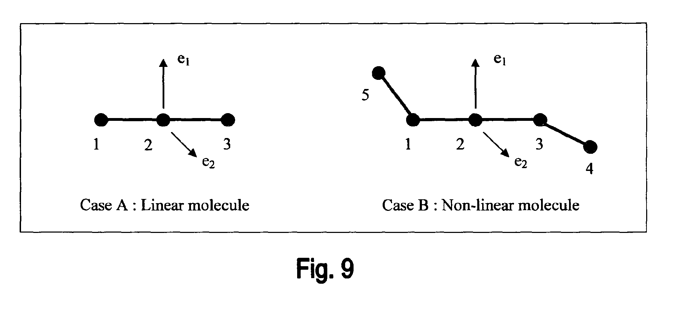

We also allow for jumps across the central atom to define additional torsion angles. In case B of Fig. 9, for instance, we would define a torsion angle that involves the atoms 5-1-3-4. Fig. 9 shows three atoms on a straight line.
In the case of linear molecules, the two reference directions $\vec{e}_1$ and $\vec{e}_2$ are determined once and for all when the delocalized internal coordinates are initialized and held fixed with respect to the molecular reference frame afterwards. In principle, we could do the same for non-linear molecules. However, it may happen that one of the two space fixed reference directions progressively becomes parallel to the axis running through the atoms 1 and 3 upon structure optimization. In such a case, the deviation from a straight line again becomes ill-defined. It is therefore more appropriate to chose reference directions related to the atomic coordinates. With respect to case B of Fig. 9, for instances, we could have chosen the following atom related reference directions:

$$\vec{e}_1 = (\vec{x}_5 - \vec{x}_1) - \frac{((\vec{x}_5 - \vec{x}_1)^T (\vec{x}_1 - \vec{x}_2))}{|\vec{x}_1 - \vec{x}_2|} \frac{(\vec{x}_1 - \vec{x}_2)}{|\vec{x}_1 - \vec{x}_2|} \qquad \text{(Eq. 5.4.5.e2)}$$

$$\vec{e}_2 = \vec{e}_1 \times (\vec{x}_1 - \vec{x}_2) \qquad \text{(Eq. 5.4.5.f2)}$$

The first derivatives of $\tilde{\varphi}_1$ and $\tilde{\varphi}_2$ with respect to the atomic coordinates can again be calculated using standard derivation rules. The generalization to more than 3 atoms on a straight line is straight forward.
It is important to realize that the coordinate transformation from delocalized internal coordinates to Cartesian coordinates, initialized for a certain starting geometry, may not be valid for all conformations of the molecule. Because of the curvilinear nature of the delocalized internal coordinates, there may not be a one to one correspondence with the Cartesian coordinate system and it may happen that the iterative procedure used for the coordinate transformation does not converge if one moves too far away from the starting point. In addition, certain bond angles, initially not close to $\pi$, may approach $\pi$ and vice versa, requiring a redefinition of the set of internal coordinates used to define the delocalized internal coordinates. During an energy minimization, it is important to monitor if the transformation from delocalized internal coordinates to Cartesian coordinates is still well defined. If the transformation is no longer valid, the minimization needs to be stopped and the coordinate transformation has to be reinitialized using the currently best structure as the new initial geometry. Then, the minimization procedure can be restarted.
In this section, we have introduced two significant advancements compared to the approach described by Baker et al. Firstly we have reformulated the approach of Baker et al in such a way that the molecular point group symmetry can be explicitly taken into account, resulting in an important reduction of the total number of degrees of freedom. Secondly, we have addressed and solved the problem of ill-defined whole molecule translations and rotations, making it possible to use delocalized internal coordinates not only for isolated molecules but also for molecules in a crystalline environment.

**5.4.6 From relative coordinates to natural coordinates and back - a summary**

In subsection 5.4.3 to subsection 5.3.5, we have defined the natural coordinate system and we have described the various steps required to transform natural coordinates to fractional coordinates. As the great amount of mathematical detail presented in the previous subsections may hamper the understanding of the overall procedure, we summarize the most important steps in this subsection.
The use of natural coordinates for lattice energy minimization implies three distinct tasks: the initialization (one may also say the definition) of the natural coordinate system, the transformation from natural coordinates to fractional coordinates and the transformation from first energy derivatives in fractional coordinates to first energy derivatives in natural coordinates. In this subsection, we take a second look at the first two tasks. The transformation of energy derivatives is described in the subsequent subsection.

In most databases, scientific publications or program outputs, crystal structures are defined in terms of lattice parameters and fractional atomic coordinates. The initialization of the natural coordinate system consists of the determination of a certain number of scalars, vectors and matrices that relate the natural coordinate system to the fractional coordinate system. A list of the required scalars, vectors and matrices is presented in Tab. 5.4.6.a.

The transformation from the natural coordinate system to the fractional coordinate system involves the following steps. First new lattice vectors $\vec{a}$, $\vec{b}$ and $\vec{c}$ and a new transformation matrix $\bar{L}$ are determined using (Eq. 5.4.3.a), (Eq. 5.4.3.b) and (Eq. 5.4.3.i). The lattice parameters $c_i$ used in the fractional coordinate system can be obtained from the lattice vectors $\vec{a}$, $\vec{b}$ and $\vec{c}$ by means of (Eq. 5.4.3.c)- (Eq. 5.4.3.h) and Tab. 5.1.a.

In a second step, all independent molecules must be dealt with one by one. First, the delocalized internal coordinates $\theta_1, \ldots \theta_{n\theta}$ must be converted to Cartesian displacements $\vec{\zeta}$ using the iterative procedure described in subsection 5.4.5. The Cartesian displacements can be converted to uncorrected Cartesian coordinates using (Eq. 5.4.5.k). Then, corrected Cartesian coordinates in the molecular reference frame must be determined using (Eq. 5.4.5.1) - (Eq. 5.4.5.o) and the iterative procedure for the calculation of $\bar{R}_{corr}$ ((Eq. 5.4.5.p)- (Eq. 5.4.5.t)). Finally, after having determined the whole molecule translation from (Eq. 5.4.4.e) and the whole molecule rotation from (Eq. 5.4.4.d), the atomic positions $\bar{v}_i$ used in the fractional coordinate system can obtained from (Eq. 5.4.4.k).

Tab. 5.4.6.a: Scalars, vectors and matrices that are determined when the natural coordinate system is initialized. Objects referring to molecular properties need to be defined for each molecule.

| Symbol | Description |
|---|---|
| $\overline{L}_0$ | Initial transformation matrix from fractional atomic coordinates to the external Cartesian coordinate system |
| $\widetilde{\overline{M}}_i$ | Matrices defining symmetry allowed lattice deformations |
| cry(i,j) | Lookup table that relates atom i in the asymmetric unit of molecule j to the corresponding atom in the asymmetric unit of the crystal |
| mol(i) & at (i) | Lookup tables that relates the atom i in the asymmetric unit of a crystal to the corresponding atom in the asymmetric unit of a molecule |
| $\widetilde{\overline{S}}_i \& \widetilde{\overline{s}}_i$ | Symmetry operations relating the atoms in the asymmetric unit of the crystal to the corresponding atoms in a molecule |
| $\overline{\Gamma}_{i,j}$ | Molecular point group symmetry operations generating symmetry copy j of atom i |
| $\vec{T}_0$ | Molecular centre in fractional coordinates |
| $\vec{\xi}_i$ | Symmetry allowed molecular move directions in fractional coordinates |
| $\overline{R}_0$ | Transformation matrix from molecular Cartesian coordinate system to the shifted external Cartesian coordinate system |
| $\overline{R}_i$ | Symmetry allowed rotation matrices |
| $\vec{\vartheta}_{i,0}$ | Initial atomic coordinates in the molecular Cartesian coordinate system |
| $\vec{\upsilon}_{i,j}$ | Symmetry allowed atomic move directions in Cartesian coordinates |
| - | Complete list of internal coordinates (bonds lengths, bond angles, etc.) |
| $\vec{q}_0$ | Initial values of the internal coordinates |
| $\vec{\psi}_i$ | Vectors defining a non-redundant set of delocalized internal coordinates |
| $\vec{\vartheta}'_{corr}$ | Translation that insures that the molecular centre coincides with the origin of the molecular coordinate system. Initially zero. |
| $\varphi_i$ | Rotation angles that insure that the molecule adopts a well defined orientation in the molecular coordinate system. Initially zero. |

### 5.4.7 Energy derivatives with respect to natural coordinates

In section 5.2 and section 5.3 we have seen how energies and energy derivatives can be calculated using the fractional coordinate system. In this subsection, we describe how energy derivatives the fractional coordinate system can be converted to energy derivatives in the natural coordinate system.

To simplify the general part of the discussion, we define a vector $\phi$ that comprises all $n\phi$ coordinates of the fractional coordinate system (cell parameters c; and relevant components of the atomic positions $v_i$) and a vector $\eta = (\lambda_0, ..., \lambda_{n\lambda}, \tau_{1,1}, ... \tau_{n\tau1,1}, \rho_{1,1}, ... \rho_{n\rho1,1}, \theta_{1,1}, ... \theta_{n\theta1,1}\tau_{1,2}, ...)$ that comprises all $n\eta$ natural coordinates. Using the chain rule for derivatives, one obtains the first energy derivatives with respect to natural coordinates:

$$\frac{\partial E}{\partial \eta} = \overline{\Omega}^T \frac{\partial E}{\partial \vec{\phi}} \qquad \text{(Eq. 5.4.6.a)}$$

Here $\partial E / \partial \vec{\phi}$ is known and $\overline{\Omega}$ is defined below:

$$\overline{\Omega} = \begin{pmatrix} \partial\phi_1/\partial\eta_1 & \cdots & \partial\phi_1/\partial\eta_{n\eta} \\ \vdots & \ddots & \vdots \\ \partial\phi_{n\phi}/\partial\eta_1 & \cdots & \partial\phi_{n\phi}/\partial\eta_{n\eta} \end{pmatrix}$$ (Eq. 5.4.6.b)

In principle, the derivatives $\partial_i / \partial\eta_j$ can be obtained by applying standard derivation rules to the equations in subsection 5.4.3, 5.4.4 and 5.4.5. We therefore only discuss some aspects of the derivative calculation.

The lattice parameters $c_i$ of the fractional coordinate system only depend on the parameters $\lambda_i$ of the natural coordinates system. The relationship between the two sets of parameters can be established via Tab. 5.1.a, (Eq. 5.4.3.c)- (Eq. 5.4.3.h), (Eq. 5.4.3.a) and (Eq. 5.4.3.i).

The atomic coordinates $\vec{v}_i$ depend on all natural coordinates. The central equation for the transformation from natural coordinates to atomic coordinates $\vec{v}_i$ is (Eq. 5.4.4.k). $\bar{L}^{-1}$ is the only term in (Eq. 5.4.4.k) that depends on the natural coordinates $\lambda_0, ..., \lambda_{n\lambda}$. $\partial\bar{L}^{-1}/\partial\lambda_i$ is related to $\partial\bar{L}/\partial\lambda_i$ via:

$$\frac{\partial\bar{L}^{-1}}{\partial\lambda_i} = -\bar{L}^{-1}\frac{\partial\bar{L}}{\partial\lambda_i}\bar{L}^{-1}$$ (Eq. 5.4.6.c)

$\bar{L}$ depends on $\lambda_o, ..., \lambda_{n\lambda}$ via (Eq. 5.4.3.b) and (Eq. 5.4.3.i). $T_{mol(i)}$ is the only term in (Eq. 5.4.4.k) to depend on the natural coordinates $\tau_1, mol(i), ... \tau_{n\tau}, mol(i)$ via (Eq. 5.4.4.e) and $\bar{R}_{mol(i)}$ is the only term to depend on the natural coordinates $\rho_1, mol(i), ... \rho_{n\rho}, mol(i)$ via (Eq. 5.4.4.d). Finally, $\vartheta_{at(i),mol(i)}$ is the only term that depends on the delocalized internal coordinates $\theta_1, mol(i), ... \theta_{n\theta1}, moi(i)$. Knowing the derivatives of $\bar{L}$, $T_{mol(i)}$, $\bar{R}_{mol(i)}$ and $\vartheta_{at(i),mol(i)}$ with respect to the natural coordinates, the derivatives of $v_i$ with respect to the natural coordinates are readily obtained by the application of standard derivation rules to (Eq. 5.4.4.k).

The calculation of the derivatives of $\vec{\vartheta}_i$ (Here we have dropped the index mol(i) and we have renamed at(i) to i in order to simplify the succeeding expressions) with respect to $\theta_j$ is a little bit more complicated than the calculation of the other derivatives. The corrected Cartesian coordinates $\vec{\vartheta}_i$ are related to the uncorrected Cartesian coordinates $\vec{\vartheta}_i$, via (Eq. 5.4.5.1). We thus obtain:

$$\frac{\partial\vec{\vartheta}_i}{\partial\theta_j} = \frac{\partial\bar{R}_{corr}}{\partial\theta_j}(\vec{\vartheta}_i' - \vec{\vartheta}_{corr}') + \bar{R}_{corr}\frac{\partial\vec{\vartheta}_i'}{\partial\theta_j}$$ (Eq. 5.4.6.d)

Taking into account (Eq. 5.4.5.h), (Eq. 5.4.5.i) and (Eq. 5.4.5.k) $\partial\vec{\vartheta}_i / \partial\theta_j$ is straight forward to calculate. Expanding the left side of (Eq. 5.4.5.o) into a Taylor series up to first order, one obtains:

$$\frac{\partial\bar{R}_{corr}}{\partial\theta_j} = \sum_{i=1}^{n\rho}\frac{\partial\bar{R}_{corr}}{\partial\varphi_i}\frac{\partial\varphi_i}{\partial\theta_j}$$ (Eq. 5.4.6.e)

$$\left(\frac{\partial\varphi_i}{\partial\theta_j},\cdots,\frac{\partial\varphi_{n\rho}}{\partial\theta_j}\right)^T = -\bar{M}^{-1}\vec{\tilde{m}}$$ (Eq. 5.4.6.f)

$$\vec{\tilde{m}} = (\tilde{m}_1,\ldots,\tilde{m}_{n\rho})^T$$ (Eq. 5.4.6.g)

$$\tilde{m}_k = \vec{n}_k^T \left( \sum_{i=1}^{\eta_i} \sum_{j=1}^{\mu_i} \overline{\Gamma}_{i,j} \vec{\vartheta}_{i,0} \times \overline{\Gamma}_{i,j} \overline{R}_{corr} \frac{\partial \vec{\vartheta}_i'}{\partial \theta_j} \right)$$

(Eq. 5.4.6.h)

The matrix $\bar{M}$ is defined in (Eq. 5.4.5.s) and (Eq. 5.4.5.t). With $\partial \vartheta_i' / \partial \theta$ and $\partial \bar{R}_{corr} / \partial \theta_j$ known, (Eq. 5.4.6.d) can be used to calculate $\partial \vartheta_i / \partial \theta_j$.

In this section, we have dealt with the calculation of first lattice energy derivatives. If required, second derivatives can be obtained in a similar fashion.

## 5.5 Refinement of empirical parameters for the hybrid method

### 5.5.1 Deviation function

To optimize the empirical parameters used with the hybrid method, we need to define a function $F(p_1, ..., p_n)$ that depends on the empirical parameters $p_i$ and that reaches the global minimum when a good fit with the experimental data is obtained. Once such a function is defined, suitable empirical parameters can be obtained, at least in principle, by minimization of this function. In the following, we will call $F(p_1, ..., p_n)$ the deviation function and construct it un such a way that it is zero if all experimental data are precisely matched and bigger than zero otherwise.

Since we want to adjust the empirical parameters to low temperature crystal structures, we need to quantify the deviation between a set of experimental crystal structures and a corresponding set of calculated crystal structures. The empirical potentials mainly affect the unit cell parameters and the molecular arrangement in the unit cell, while they have little effect on the molecular conformations. It is therefore appropriate to focus on the comparison of unit cell parameters.

Given two unit cells with unit cell vectors $a_1$, $b_1$, $c_1$ and $a_2$ $b_2$, $c_2$ we calculate the transformation matrix $T_{1 \rightarrow 2}$ from one set of unit cell vectors to the other:

$$\vec{a}_2 = \bar{T}_{1 \rightarrow 2} \vec{a}_1, \ \vec{b}_2 = \bar{T}_{1 \rightarrow 2} \vec{b}_1, \ \vec{c}_2 = \bar{T}_{1 \rightarrow 2} \vec{c}_1$$

(Eq. 5.5.1.a)

The transformation matrix can be determined from the two matrices $L_1$ and $L_2$ the columns of which are identical to the unit cell vectors:

$$\bar{L}_i = (\vec{a}_i, \vec{b}_i, \vec{c}_i)$$

(eq. 5.5.1.b)

$$\bar{T}_{1 \rightarrow 2} = \bar{L}_2 \bar{L}_1^{-1}$$

(Eq. 5.5.1.c)

According to the linear algebra theorem on singular value decomposition, any square matrix can be written as the product of an orthonormal matrix $\bar{U}_1$ a diagonal matrix $\bar{D}$ and another orthonormal matrix $\bar{U}_2$:

$$\bar{T}_{1 \rightarrow 2} = \bar{U}_1 \bar{D} \bar{U}_2 = \bar{T}_{rot} \bar{T}_{def}$$

(Eq. 5.5.1.d)

$$\bar{T}_{rot} = \bar{U}_1 \bar{U}_2$$

(Eq. 5.5.1.e)

$$\bar{T}_{def} = \bar{U}_2^{-1} \bar{D} \bar{U}_2$$

(Eq. 5.5.1.f)

$\bar{T}_{def}$ can be understood as a compression/expansion of the original lattice along three perpendicular directions. The directions are given by the rows of $\bar{U}_2$, while the size of the compression/expansion is given by the diagonal elements of $\bar{D}$. The matrix $\bar{T}_{rot}$ is a rotation matrix. The transformation $\bar{T}_{1 \rightarrow 2}$ thus consists of a deformation followed by a rotation. Only the deformation changes the geometry of the unit cell and is therefore relevant for the construction of the deformation function F. To characterize the overall deformation by a single value, we use the following two expressions:

$$\Delta_I = \sum_{i=1}^{3} \frac{1}{2}\left(\left|d_i - 1\right| + \left|\frac{1}{d_i} - 1\right|\right) \qquad \text{(Eq. 5.5.1.g)}$$

$$\Delta_{II} = \sum_{i=1}^{3} \frac{1}{2}\left((d_i - 1)^2 + \left(\frac{1}{d_i} - 1\right)^2\right) \qquad \text{(Eq. 5.5.1.h)}$$

Here $d_1$, $d_2$ and $d_3$ are the diagonal elements of $\bar{D}$. Both expressions are symmetric with respect to $d_i$ and $1/d_i$ to insure that the transformations $\bar{T}_{1\to2}$ and $\bar{T}_{2\to1}$ are attributed the same value. (Eq. 5.5.1.h) is more appropriate for parameter optimization, since it allows for continuous first and second derivatives. (Eq. 5.5.1.g) results in discontinuous derivatives whenever it reaches zero, but the physical meaning of this expression is easier to understand. For small deformations, (Eq. 5 .5.1.g) reduces to:

$$\Delta_I = \sum_{i=1}^{3} \left|d_i - 1\right| \qquad \text{(Eq. 5.5.1.i)}$$

In the absence of any lattice deformation, all $d_i$ equal one and $\Delta_I$ is zero. If the lattice is compressed/expanded by 1% along a single direction, $\Delta_I$ equals 1%. If the lattice is compressed/expanded by 1% along all three perpendicular directions, $\Delta_I$ equals 3%. $\Delta_I$ thus is the sum of the absolute values of the individual compressions/expansions.

The deviation function for a set of N crystal structures is given by the following expression:

$$F = \sum_{j=1}^{N} \frac{1}{N} \frac{\Delta_{II,j}}{w_j^2} \qquad \text{(Eq. 5.5.1.j)}$$

Here $\Delta_{II,j}$ is the deviation between the j-th experimental crystal structure and the corresponding result of a lattice energy minimization. $w_j^2$ is a weighting factor. To compute F for a given set of empirical parameters, one has to minimize the lattice energy and evaluate (Eq. 5.5.1.h) for each member in a set of N crystal structures.

It is easy to generalize (Eq. 5.5.1.j) so that it takes into account additional experimental information. If a crystal structure A is known to be more stable than another crystal structure B of the same molecule, one may add a term that contains a deviation of the following type:

$$\Delta_{E,AB} = \begin{cases} (E_A - E_B)^2 & E_A \geq E_B \\ 0 & E_A < E_B \end{cases} \qquad \text{(Eq. 5.5.1.k)}$$

Here $E_A$ and $E_B$ are the lattice energies per molecule of the two crystal structures. For the parameter refinement presented in subsection 5.5.4, we have only used structural information.

### 5.5.2 Optimization procedure

In principle, one could minimize the deviation function F directly with a standard algorithm such as the Powell minimization algorithm. In practice, however, this straight forward approach turns out to inconvenient. At each function evaluation, all crystal structures need to be re-optimized and the calculation times for the lattice energy minimization of a single crystal structure range from several hours to several days on a state-of-the-art PC. Even if the calculations are carried out on a PC cluster, the CPU times involved get rapidly out of hand.

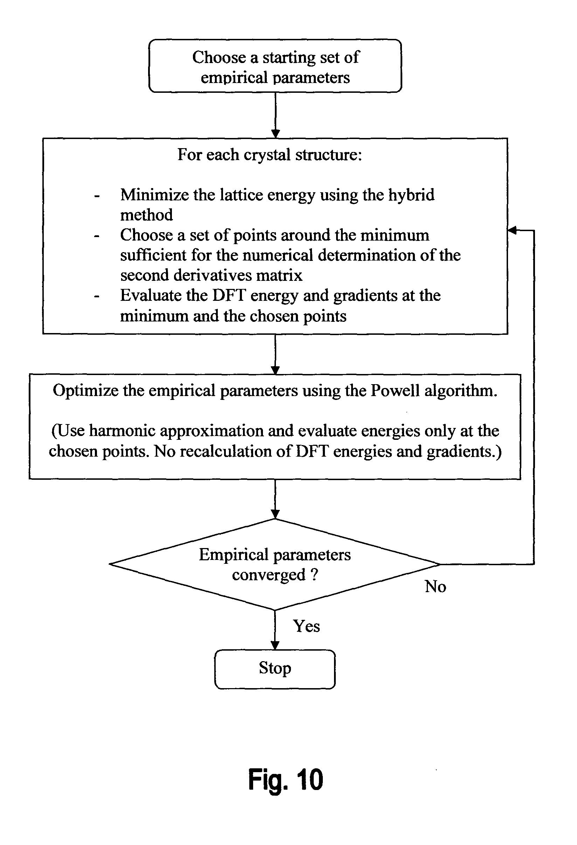

To cut down on calculation times, we use an iterative procedure to exploit the fact that the energies and forces from the time consuming DFT part of the calculation do not change when the empirical parameters are modified (see Fig. 10). Fig. 10 shows a flow diagram for parameter refinement. Starting from an initial set of reasonable empirical parameters, all crystal structures are optimized. For each crystal structure, a set of points is chosen around the lattice energy minimum. DFT energies and gradients are calculated at all these points, which have to be chosen in such a way that the second derivatives (Hessian) matrix can be constructed from the gradients.

In a second step, the empirical parameters are optimized without performing any additional DFT calculations. The Powell algorithm [Press et al 2002] used for the parameter optimization does not require any derivative information. Each time the deviation function F needs to be evaluated for a new set of empirical parameters, we determine the new calculated crystal structures in the harmonic approximation using the stored energy and gradient information. For each crystal structure, we calculate the VdW energies and gradients at the chosen points and combine them with the stored DFT energies and gradients to get the total energies and gradients. The later are used to determine the second derivatives matrix. Using the second derivatives matrix in conjunction with the energy and the gradient at the central reference position, the new lattice energy minimum is determined approximately.

The Powell algorithm returns an improved set of empirical parameters that is used as a starting point for the next iteration which begins with the lattice energy minimization of each crystal structure. The iterative procedure is stopped when sufficient convergence of the empirical parameters is achieved. In the following paragraphs, we discuss some aspects of the optimization procedure in more detail.

At each step of the iterative procedure, the initial crystal structure optimizations are carried out with fully flexible molecules. From then on, all molecules are considered to be rigid to reduce the number of structural degrees of freedom.

We now describe how the lattice energy minimum of a crystal structure can be determined in the harmonic approximation. Let $q_{min} = (q_{min,0}, ..., q_{mam})$ be the vector of m coordinates (unit cell deformations, whole molecule translations and whole molecule rotations) that corresponds to the lattice energy minimum obtained for empirical parameters $\bar{p} = (p_1, ...,p_n)$. Around the minimum $\vec{q}_{min}$, we chose m points $\vec{q}_{+,i}$ and m points $\vec{q}_{-,i}$:

$$\vec{q}_{+,i} = \vec{q}_{min} + \Delta q_i \ast \vec{e}_i \tag{Eq. 5.5.2.a}$$

$$\vec{q}_{-,i} = \vec{q}_{min} - \Delta q_i \ast \vec{e}_i \tag{Eq. 5.5.2.b}$$

Here $\vec{e}_i$ is the unit vector for which all elements except for the i-th element are zero. For each i, the scalar value $\Delta q_i$ is chosen such that:

$$E_{pot,p}(\vec{q}_{+,i}) - E_{pot,p}(\vec{q}_{min}) \cong E_{pot,p}(\vec{q}_{-,i}) - E_{pot,p}(\vec{q}_{min}) \cong \Delta E_{target} \tag{Eq. 5.5.2.c}$$

In the above equation, the index **p** refers to the empirical parameters $\bar{p} = (p_1, ...,p_n)$. $\Delta E_{target}$ is a constant used in the calculation. A value of $\Delta E_{target} = 0.25$ kcal/mol has turned out to be appropriate in practice. The use of a target energy $\Delta E_{target}$ insures that the points $\vec{q}_{+,i}$ and $\vec{q}_{-,i}$ are positioned at the right distance from the minimum: within the potential energy well of the local minimum, but far away enough to avoid problems related to the noise level of the energy calculation. For later use, we calculate and store the gradients $\nabla E_{DFT}(q_{+i})$, $\nabla E_{DFT}(\vec{q}_{-,i})$, $\nabla E_{EDIT}(\vec{q}_{min})$ and the energy $E_{DIT}(\vec{q}_{min})$.

Now let us suppose that we would like to know the minimum of the lattice energy for a new set $\vec{p}' = (p'_1, ..., p'_n)$ of empirical parameters. We can obtain the following energy and gradients without any further DFT calculations:

$$E_{pot,p'}(\vec{q}_{min}) = E_{DFT}(\vec{q}_{min}) + E_{VdW,p'}(\vec{q}_{min}) \tag{Eq. 5.5.2.d}$$

$$\nabla E_{pot,p'}(\vec{q}_{min}) = \nabla E_{DFT}(\vec{q}_{min}) + \nabla E_{VdW,p'}(\vec{q}_{min}) \tag{Eq. 5.5.2.e}$$

$$\nabla E_{pot,p'} (\vec{q}_{+,i}) = \nabla E_{DFT} (\vec{q}_{+,i}) + \nabla E_{VdW,p'} (\vec{q}_{+,i}) \text{ for } 0<i<m \qquad \text{(Eq. 5.5.2.f)}$$

$$\nabla E_{pot,p'} (\vec{q}_{-,i}) = \nabla E_{DFT} (\vec{q}_{-,i}) + \nabla E_{vdW,p'} (\vec{q}_{-,i}) \text{ for } 0<i<m \qquad \text{(Eq. 5.5.2.g)}$$

(Eq. 5.5.2.f) and (Eq. 5.5.2.g) can be used to construct the second derivatives matrix $\overline{H}$. We first construct the matrix $\overline{M}$ the columns of which are given by:

$$\text{i-th column of } \overline{M} = (\nabla E_{pot,p'} (\vec{q}_{+,i}) - \nabla E_{pot,p'} (\vec{q}_{-,i}))/2/\Delta q_i \qquad \text{(Eq. 5.5.2.h)}$$

For infinitely small $\Delta q_i$ and in the absence of rounding errors, the matrix M is equal to the second derivatives matrix. Since we use finite values of $\Delta q_i$, this relationship holds only approximately. Unlike the second derivatives matrix $\overline{H}$, $\overline{M}$ *is* not exactly symmetric in most cases. We therefore use the following expression to obtain a symmetric approximate second derivatives matrix:

$$\overline{H} \cong 0.5^*(\overline{M}+\overline{M}^T) \qquad \text{(Eq. 5.5.2.i)}$$

Here $\overline{M}^T$ is the transpose of $\overline{M}$. In the harmonic approximation, the new lattice energy minimum $q'_{min}$ is given by the following expression:

$$\vec{q}'_{min} = \vec{q}_{min} - \overline{H}^{-1} \nabla E_{pot,p'} (\vec{q}_{min}) \qquad \text{(Eq. 5.5.2.j)}$$

$\overline{H}^{-1}$ is the inverse matrix of $\overline{H}$. The lattice energy at the new minimum is:

$$E_{pot,p'} (\vec{q}_{min}) = E_{pot,p'} (\vec{q}_{min}) + \nabla E_{pot,p'} (\vec{q}_{min})^T \Delta\vec{q} + 0.5^* \Delta\vec{q}^T \overline{H} \Delta\vec{q}$$

$$\text{with } \Delta\vec{q} = \vec{q}'_{min} - \vec{q}_{min} \qquad \text{(Eq. 5.5.2.k)}$$

### 5.5.3 Starting values for the form factor n, the damping radii r and the $C_6$ coefficients

The optimization procedure described in the previous section leads to the local minimum for which the basin of attraction reaches out to the set of starting parameters. As a consequence, one can only get close to the global minimum if appropriate starting parameters are chosen. The use of more sophisticated global optimization methods that do not depend on the choice of starting parameters (The method described in 5.5.2 is a local optimization method.) such as simulated annealing or parallel tempering is currently beyond reach because of the long calculation times involved. Our derivation of appropriate starting parameters closely follows the work of Wu and Yang [Wu and Yang 2002].

For the form factor n, we set n=1 as this choice corresponds to the damping function used by Wu and Yang. Starting values for the homonuclear damping radii $r_{A,A}$ are obtained by multiplying Bondi's VdW radii [Bondi 1964] by a factor of two. All atoms of the same element are attributed the same starting value, regardless of their chemical environment. Starting values for heteronuclear damping radii $r_{A,B}$ are generated using an arithmetic combination rule:

$$r_{A,B} = \frac{1}{2} (r_{A,A} + r_{B,B}) \qquad \text{(Eq. 5.5.3.a)}$$

Values for the $C_6$ coefficients can be obtained by fitting atomic $C_6$ coefficients to molecular $C_6$ coefficients from dipole oscillator strengths distributions [Wu and Yang 2002]. According to Wu and Yang, the atomic $C_6$ coefficients significantly depend on the hybridisation state of the atom. We use the hybridization dependent $C_6$ coefficients proposed by these authors. $C_6$ coefficients for unlike atom pairs can be obtained from the $C_6$ coefficients of like atom pairs using the following combination rule:

$$C_{6,A,B} = \frac{2(C_{6,A,A}^2 C_{6,B,B}^2 N_{eff,A} N_{eff,B})^{1/3}}{(C_{6,A,A} N_{eff,B}^2)^{1/3} + (C_{6,B,B} N_{eff,A}^2)^{1/3}} \qquad \text{(Eq. 5.5.3.b)}$$

Here $N_{eff}$ is the effective electron number. Like Wu and Yang, we use the effective electron numbers proposed by Halgren [Halgren 1992].

The various parameters mentioned above are summarized in Tab. 5.5.3.a for the different hybridization depend atom types of hydrogen, carbon, oxygen and nitrogen. For nitrogen, we use a single atom type because the available experimental information does not allow for the refinement of hybridization dependent $C_6$ coefficients in this case. For carbon and oxygen, we derive the hybridization state from the number of covalently bonded neighbours.

It is important to stress that the approach presented in this document is not limited to hydrogen, carbon, oxygen and nitrogen. VdW radii and effective electron numbers are readily available for many elements and molecular $C_6$ coefficients have been published for molecules containing elements such as F, Cl, Br and S.

Tab. 5.5.3.a:

| Starting parameters for hydrogen, carbon, nitrogen and oxygen. | | | |
|---|---|---|---|
| Atom type A | $C_{6,A,A}$ [Å$^6$kcal/mol] | $N_{eff,A}$ | $r_{A,A}$ [Å] |
| H | 38.93 | 0.80 | 2.40 |
| C(sp$^3$) | 303.8 | 2.49 | 3.40 |
| C(sp$^2$) | 376.6 | 2.49 | 3.40 |
| C(sp) | 409.8 | 2.49 | 3.40 |
| N | 265.8 | 2.82 | 3.10 |
| O(sp$^3$) | 159.9 | 3.15 | 3.04 |
| O(sp$^2$) | 178.3 | 3.15 | 3.04 |

The technique for the derivation of molecular $C_6$ coefficients from oscillator strength distributions is a mixture of experimental and theoretical components. Even though not completely based on experimental data alone, it is likely that the molecular $C_6$ coefficients used by Wu and Yang are fairly accurate. As their atomic $C_6$ coefficients reproduce the molecular data extremely well, it can be assumed that the atomic $C_6$ coefficients accurately model the Van der Waals interaction at long interatomic distances. The $C_6$ coefficients in Tab. 5.5.3.a are thus much more than just reasonable starting values and further refmement of these values is not necessarily required. In other words, the long range part of the empirical pair potentials can be determined independently of the exact nature of the DFT part of the hybrid method. The damping radii and the form factor, on the other hand, determine the behaviour of the empirical pair potentials at short to intermediate interatomic distances where the DFT part and the VdW part of the hybrid method are both important. They <u>must</u> be adjusted such that the hybrid method as a whole accurately reproduced experimental results.

### 5.5.4 Preliminary parameter refinement for C, N, O and H

Ultimately the approach described in this document is expected to work for a wide variety of elements. At the point in time when this document is written, however, only a preliminary parameter refinement for C, N, O and H has been carried out and a more advanced parameter refinement including the additional elements Cl, F and S is in progress. In this section we describe the results of the preliminary parameter refinement for C, N, O and H.

Tab. 5.5.4.a: Set of crystal structures used for parameter refinement.

| Compound | Ref. | Composition | Temp. [K] | Vol. [Å3] | Dev. [%] |
|---|---|---|---|---|---|
| benzene | BENZEN06 | C6H6 | 15 | 463 | 2.2 |
| butane | Refson et al 1986 | C4H10 | 5 | 223 | 1.4 |
| propane | Boese et al 1999 | C3H8 | 30 | 365 | 2.3 |
| durene | Neumann et al 1999 | C10H14 | 1.5 | 403 | 4.0 |
| p-xylene | Prager et al 1991 | C8H10 | 4 | 311 | 1.3 |
| acetylene | McMullan et al 1992 | C2H2 | 15 | 208 | 5.0 |
| acetylene cubic | McMullan et al 1992 | C2H2 | 143→0 | 216 | 0.9 |
| acetic acid | ACETAC06 | C2H4O2 | 4 | 289 | 2.2 |
| formic acid | FORMAC02 | CH2O2 | 5 | 193 | 1.6 |
| furan | BUNJAV02 | C4H8O | 5 | 401 | 2.5 |
| methanol | METHOL02 | CH4O | 15 | 201 | 7.5 |
| terephtalic acid | TEPHTH03 | C8H6O4 | 2 | 169 | 3.9 |
| hexamethylenetetramine | HXMTAM10 | $C_6H_{12}N_4$ | 15 | 332 | 1.3 |
| nitrogen alpha | Mills et al 1986 | $N_2$ | 20 | 181 | 1.9 |
| nitrogen gamma | Mills et al 1986 | $N_2$ | 20 | 80 | 2.1 |
| nitromethane | NTROMA13 | $CH_3NO_2$ | 4 | 275 | 3.3 |
| N,N'-diformohydrazide | FOMHAZ16 | $C_2H_4N_2O_2$ | 15 | 178 | 2.4 |
| N-hydroxy-methaneimidamide | FORAMO01 | $CH_4N_2O$ | 16 | 277 | 9.8 |
| glyoxime | GLOXIM11 | $C_2H_4N_2O_2$ | 9 | 178.6 | 6.2 |
| urea | UREAXX12 | $CH_4N_2O$ | 12 | 145 | 3.4 |

To refme the empirical parameters used with the hybrid method, a set of 20 low temperature crystal structures was selected which is listed in Tab. 5.5.4.a. The table presents the compound name, the CSD reference code or a literature reference for the crystal structure, the composition of the compound, the experimental temperature and the volume of the unit cell. The cell parameters of the cubic phase of acetylene were extrapolated down to 0K using cell parameters from a series of measurements at 143 K and above. All crystal structures were measured at normal pressure apart from the crystal structure of γ-nitrogen determined at a pressure of 407 MPa. The crystal structures listed in Tab. 5.5.4.a correspond to a total number of 62 variable unit cell parameters.

In a first refinement, only five parameters were adjusted, namely the form factor n and one damping radius per element. The $C_6$ coefficients were kept constant at the values indicated in Tab. 5.5.3.a. The combination rules (Eq. 5.5.3.a) and (Eq. 5.5.3.b) were used to obtain damping radii and $C_6$ coefficients for pairs of unlike atoms. Following the procedure outline in section 5.5.2, the five parameters were adjusted to reproduce the unit cells of the experimental crystal structures, i.e. to minimize (Eq. 5.5.1 j) with $w_i$ = 0.001. After two iterations (see Fig. 10), the parameter refinement had essentially converged and a value F=650 was obtained for the deviation function. The individual deviations $\Delta_l$ (see (Eq. 5.5.1.g)) of the calculate crystal structures from the experimental crystal structures after parameter refinement are shown in the last column of Tab. 5.5.4.a. On average, calculated and experimental crystal structures deviate by $\bar{\Delta}_l$ =3.3%, corresponding to average cell lengths errors of roughly 1%. In summary it can be said that the adjustment of only 5 parameters yields a very satisfying agreement between the calculated and the experimental crystal structures. The values of the adjusted parameters before and after parameter refinement are presented in Tab. 5.5.4.b.

Tab. 5.5.4.b:

| Values before and after parameter refinement. | | |
|---|---|---|
| Parameter | before | after |
| n | 1.00 | 0.2621 |

Tab. 5.5.4.b: (continued)

| Values before and after parameter refinement. | | |
|---|---|---|
| Parameter | before | after |
| $r_{H,H}$ [Å] | 2.40 | 3.3029 |
| $r_{C,C}$ [Å] | 3.40 | 3.8140 |
| $r_{N,N}$[Å] | 3.10 | 3.3255 |
| $r_{O,O}$[Å] | 3.04 | 2.8301 |

Using the results in Tab. 5.5.4.b as a starting point, several attempts were made to further improve the agreement between the optimized and the experimental crystal structures by allowing for more degrees of freedom in the parameter refinement (hybridization dependent damping radii, individual damping radii for unlike atom pairs instead the using a combination rule, $C_6$ coefficients) and by trying different combination rules instead of (Eq. 5.5.3.a). None of these attempts led to significant improvements and many refinements resulted in unreasonable parameters for the damping radii or the $C_6$ coefficients. In general, it was found that there is a very strong correlation between the damping radii on the one hand and the form factor and the $C_6$ coefficients on the other hand. Almost the same overall agreement between the calculated and the experimental crystal structures can be obtained for different form factors and/or different sets of $C_6$ coefficients if the damping radii are adjusted independently for each of these choices. To avoid unphysical values, it is therefore advisable to us the $C_6$ coefficients from Tab. 5.5.3.a without further refinement.

It needs to be stressed that the results presented in this section are only preliminary. There may be other damping functions, combination rules or sets of adjustable parameters that yield a significantly better agreement between the calculated and the experimental crystal structures. A more thorough investigation of the various options is currently under way. It is also important to note that parameters in Tab. 5.5.4.b are only guaranteed to yield accurate results in conjunctions with the DFT method described in 5.2 that was used for the parameter refinement. If the hybrid approach is to be used with another DFT method, a reparameterization of these parameters may be required.

### 5.6 Energy ranking and *in silico* polymorph screening

In the previous section, we have shown how the empirical parameters of the hybrid method can be derived from structural information. In principle, there is no guarantee that the obtained parameter set is also appropriate for the accurate energy ranking of crystal structures. In this section, we present a validation study which clearly indicates that the hybrid method, with the parameters obtained in subsection 5.5.4, offers the accuracy required for in silico polymorph screening.

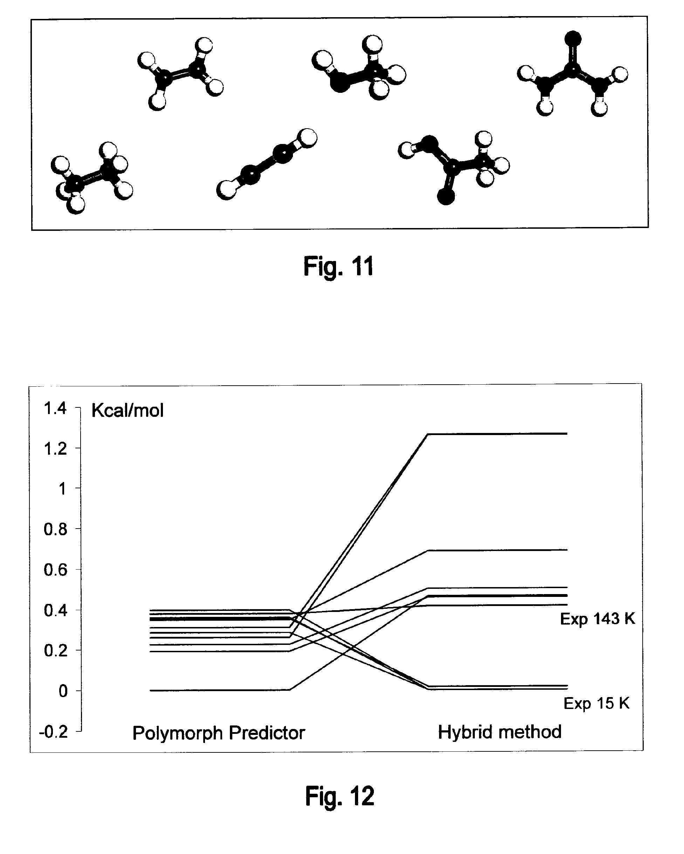

The validation study was carried out for a test set of six small molecules, namely ethane, ethylene, acetylene, methanol, acetic acid and urea (see Fig. 11). The test set covers a variety of chemical interactions. Ethane, ethylene and acetylene contain a single, double and triple carbon-carbon bond, respectively. Their crystal structures are predominantly held together by Van der Waals interactions, as these molecules cannot form hydrogen bonds and have rather small atomic partial charges. Methanol, acetic acid and urea are examples for moderately to strongly hydrogen bonded networks.

For all six molecules, the same procedure was followed. First, possible crystal packings were generated by running Accelrys's Polymorph Predictor [Accelrys 2004] twice with fine settings in the 17 most populated space groups. In five out of six cases, the Polymorph Predictor was used in conjunction with the CVFF95 [Accelrys 2004] force field which is well parameterized for many small molecules. However, CVFF95 turned out to be a poor choice for the crystal structure optimization of acetylene, and the Universal Force Field with Qeq charges [Accelrys 2004] was used instead. In a second step, the lattice energy of the 10-15 most stable crystal structures from the output of the Polymorph Predictor was re-minimized using the hybrid method and the lattice energy minimizer described in this document.

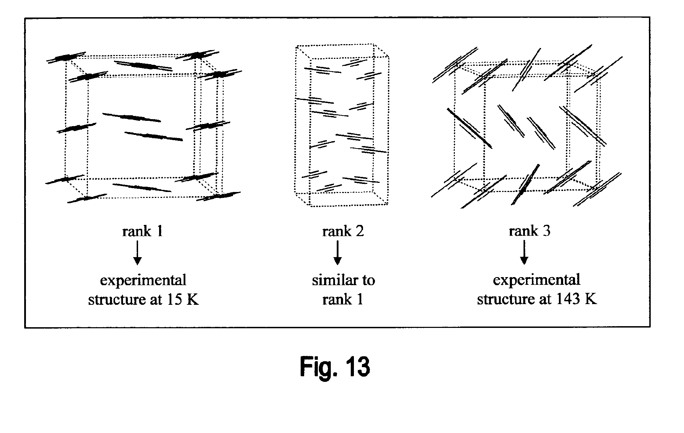

Before we present a summary of the results obtained for all six molecules, we further illustrate the approach by a more detailed discussion of the case of acetylene. Fig. 12 compares the energy ranking of the Polymorph Predictor to the energy ranking obtained with the hybrid method for the 15 most stable crystal structures found with the Polymorph Predictor (structures 11 - 15 are very similar in energy and appear as a single line). Several crystal structures proposed by the Polymorph Predictor minimize towards the same crystal structure for the hybrid method. Using the hybrid method, the low temperature crystal structure of acetylene if found as the most stable crystal structure (rank 1) and the polymorph observed experimentally at 143 K and above is found as rank 3. The intermediate crystal structure (rank 2) is closely related to rank 1. Both crystal structures consist of identical planar arrangements of molecules stacked in a similar but slightly different way. The crystal structures of rank 1, rank 2 and rank 3 are shown in Fig. 13 together with the two corresponding experimental crystal structures. The hybrid method accurately reproduces the known polymorphs of acetylene and their relative order of stability. Fig. 13 shows the three most stable crystal structures of acetylene found with the hybrid method according to the invention; for rank 1 and rank 3, a superposition with the experimental crystal structure is presented.

The excellent results obtained for acetylene are representative for the overall performance of the hybrid method. The energy ranking for all six molecules of the test set is summarized in table 5.6.a. The first three columns show the name of the compound, the temperature at which the low temperature crystal structure was determined and the name of the force field used in conjunction with the Polymorph Predictor. The forth column indicates the rank, in the output of the Polymorph Predictor, of the crystal structure that corresponds to the experimental low temperature structure. The good performance of the CVFF95 force field (rank 1 or 2 in all cases) is somewhat surprising. It illustrates that a force field, if well parameterized for a certain class of molecules, can be appropriate for in silico polymorph screening. However, force fields only work for a limited number of molecules, as illustrated by the fact that CVFF95 fails completely for acetylene. Tests for other small molecules (butane, paracetamol, etc) have shown that the Polyporph Predictor in conjunction with the CVFF95 force field typically finds the experimental crystal structure among the first 10 crystal structures. The performance observed for the test set is thus significantly better than the average performance.

Columns 5 in Tab. 5.6.a present the rank of the experimental crystal structure obtained after energy minimization with the hybrid method. Column 6 indicates the energy difference between the experimental crystal structure and rank 1. In 5 out of six cases, the experimental crystal structure corresponds to the most stable crystal structure, ethane being the only exception. Concerning the case of ethane, it has to be taken into account that the crystal structure of ethane was determined at 85 K. At this temperature, entropy effects are not negligible and it may well be that the most stable calculated crystal structure corresponds indeed the most stable crystal structure at 0 K, but not at 85 K. In addition it is important to note that the numerical accuracy of the DFT part of the calculation (see section 5.2) is only guaranteed to be better than 0.01 kcal/mol, thus being of the same order of magnitude as the energy difference between rank 1 and rank 3. Column 7 in Tab. 5.6.a indicates the number of different crystal structures found in a small energy window of 0.02 kcal/mol above the most stable crystal structure. Typically, the energy window contains more than one crystal structure - a fact with demonstrates that an accuracy of better than 0.01 kcal/mol is indeed required for the accurate energy ranking of polymorphic crystal structures.

Tab. 5.6.a:

| Energy ranking obtained for 6 small molecules with the Polymorph Predictor and with the hybrid method (see text for details). | | | | | | |
|---|---|---|---|---|---|---|
| Compound | $T_{exp}$ [K] | Force field | FF rank | Hybrid rank | Hybrid energy [kcal/mol] | Nb in window 0.00-0.02 [kcal/mol] |
| Acetylene | 15 | UFF | 12 | 1 | 0.0 | 1 |
| Ethylene | 85 | CVFF95 | 1 | 1 | 0.0 | 3 |
| Ethane | 85 | CVFF95 | 1 | 3 | 0.009 | 3 |
| Methanol | 15 | CVFF95 | 1 | 1 | 0.0 | 2 |
| Acetic acid | 4 | CVFF95 | 2 | 1 | 0.0 | 1 |
| Urea | 12 | CVFF95 | 2 | 1 | 0.0 | 2 |

The results obtained with the Polymorph Predictor and some of the figures shown in this section were kindly provided by Aventis (France) as part of a collaboration in the field of in silico polymorph screening.

In summary, it can be said that the hybrid method, with the empirical parameters derived in section 5.5.4, offers an unprecedented accuracy for the energy ranking of crystal structures.

## 5.7 The polymorphism of N2 - an interesting test case

Molecular nitrogen is an ideal test case to assess the accuracy of the hybrid method. On the one hand, the number of empirical parameters is extremely small. Indeed, we only refine the damping radius $r_{N,N}$, using the $C_6$ coefficient from Tab. 5.5.3.a and setting n = 1.0 for the form factor. On the other hand, a significant amount of experimental information is readily available [Mills et al 1986]. At low temperature, three ordered polymorphs have been observed. The $\alpha$-polymorph is the most stable structures at normal pressure. With increasing pressure, a first phase transition from the $\alpha$-polymorph to the $\gamma$-polymorph is observed at about 330 MPa. A second phase transition from the $\gamma$-polymorph

to the ε-polymorph follows at about 1.9 GPa. The crystal structures of the three polymorphs have been determined experimentally at 20K and normal pressure for the α-polymorph, at 20K and 407MPa for the γ-polymorph and at 110K and 7.8 GPa for the ε-polymorph. The availability of both structural information and energetic information (pressure dependent order of stability) provides a stringent test for the hybrid method.

Using the refinement procedure described in 5.5, we first adjust the damping radius $r_{N,N}$ such that the hybrid method reproduces the experimental crystal structures of α-$N_2$ and γ-$N_2$. A value of $r_{N,N}$ = 2.76 Å is obtained. Fig. 14 compares the calculated to the experimental crystal structures for a variety of cases. Fig. 14 shows a comparison of the experimental and calculated crystal structures of α-$N_2$ (left) and γ-$N_2$ (right) for three different calculations, each of the 6 graphics being a superposition of two crystal structures. The upper two crystal structures were obtained using the DFT method described in 5.2 without any additional empirical potentials. The two calculated unit cells are significantly larger than the two experimental unit cells, reflecting the fact the attractive long range dispersive interactions are missing. For the calculation of the next two crystal structures, the DFT calculations were combined with the empirical potentials proposed by Wu and Yang [Wu and Yang 2002]. In both cases, the calculated unit cells are smaller than the experimental unit cells, illustrating the fact that the empirical N-N potential of Wu and Yang overestimates the strengths of the empirical corrections at short and intermediate interatomic distances. The lower two crystal structures were obtained with the hybrid method described in this work after parameter refinement. The agreement is significantly better than in the first two cases.

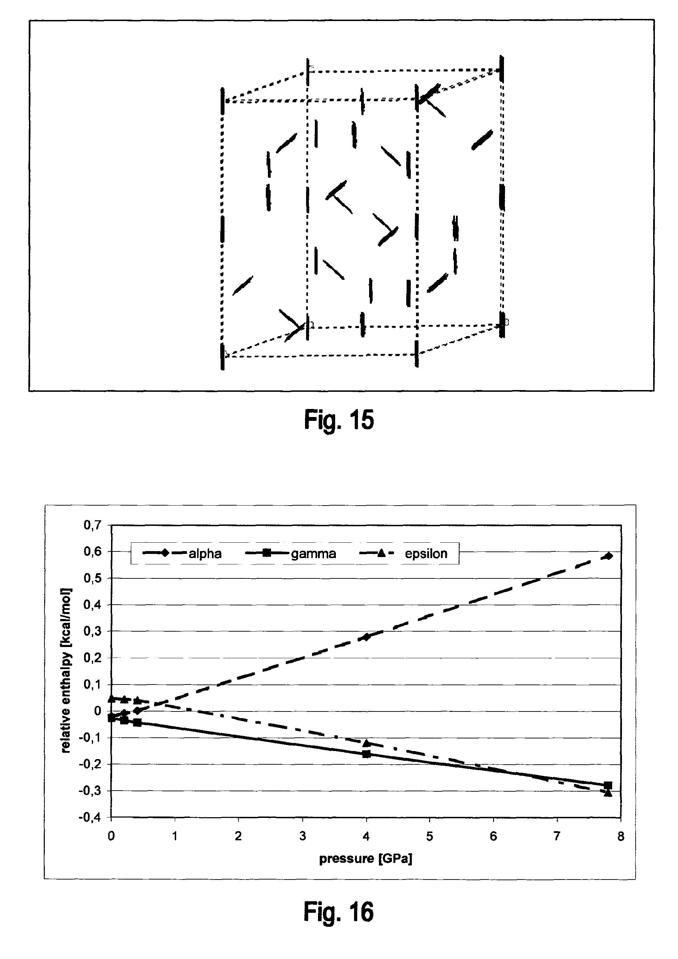

Having adjusted the damping radius $r_{N,N}$ to the crystal structures of α-$N_2$ and γ-$N_2$, we now examine how well the parameterized hybrid method performs with respect to the remaining experimental information. Fig. 15 shows a superposition of the calculated and the experimental crystal structure of ε-$N_2$. As for α-$N_2$ and γ-$N_2$, the agreement between the calculated and the experimental crystal structure is excellent.

We now turn our attention the relative stability of the three different polymorphs as a function of pressure. Lattice enthalpies were calculated for all three polymorphs at 0 MPa, 200 MPa, 407 MPa, 4 GPa and 7.8 GPa. The results are shown in Fig 16. Since the enthalpy changes as a function of pressure are significantly larger than the relative enthalpy changes, Fig. 16 shows relative enthalpies rather than absolute enthalpies. For a given pressure, the average enthalpy of the three polymorphs is set to zero. Qualitatively, the experimental order of stability is well reproduced. γ-$N_2$ is the most stable crystal structure in the intermediate pressure range. At high pressure, ε-$N_2$ is more stable than γ-$N_2$. At low temperature, enthalpy of α-$N_2$ approaches the enthalpy of γ-$N_2$. Quantitatively, however, there are some disagreements. For the two phase transitions, the calculation yields pressures of about 0 MPa and 6.5 GPa, whereas values of 330 MPa and 1.9 GPa have been observed experimentally. These disagreements are not necessary related to limitations of the hybrid method. $N_2$ is very small (light) molecule, and zero-point translations and rotations can be expected to have a significant effect on the relative enthalpies. It can be estimated that zero point effects, currently neglected in the calculation, may change the relative stabilities by about 0.05 kcal/mol. Comparing this value to the energy scale of Fig 16 it is obvious that the current neglect of zero-point effects may well be at the origin of the observed mismatch between the calculated and the experimental transition pressures.

The case of molecular nitrogen is an excellent example to illustrate the strong correlation between the damping radii and the $C_6$ coefficients that has already been mentioned in section 5.5. The parameter refinement described in this section was actually carried out for two different values of the $C_6$ coefficient. The first two lines of Tab. 5.7.a show the two values of the $C_6$ coefficient and the resulting values for the damping radius. Lines 3 to 5 show the deviations between the calculated and the experimental crystal structures for both cases. The last two lines present the enthalpy differences calculated for the two pressures at which the phase transitions occur. Ideally, these enthalpy differences should be zero. For the first $C_6$ coefficient, the parameter refinement was carried out several times with different starting points to obtain an estimate of the numerical accuracy of the fitting procedure. The corresponding error bars are indicated in Tab. 5.7.a. For the two different $C_6$ parameters, we obtain different values of the damping radii, but very similar agreement with the experimental data. Both the structural deviations and the enthalpy differences are virtually the same to within the calculated error bars.

Tab 5.7.a: Refinement of the damping radius r for two different values of the $C_6$ coefficient (see text for details).

| $C_6$ [Å6*kcal/mol] | 266 | 340 |
|---|---|---|
| r [Å] | 2.76±0.03 | 3.05 |
| Deformation α-phase [%] | 0.30±0.25 | 0.19 |
| Deformation γ-phase [%] | 1.20±0.26 | 1.54 |
| Deformation ε-phase [%] | 2.35±0.20 | 1.98 |
| $E_\alpha$-$E_\gamma$ (330 MPa) [kcal/mol] | 0.034±0.003 | 0.032 |
| $E_\gamma$-$E_\varepsilon$ (1.9 GPa) [kcal/mol] | -0.066±0.001 | -0.066 |

**References**

Accelrys Inc. (2004), 9685 Scranton Road, San Diego, CA 92121-3752, USA

Andzelm J. et al (2001), Chem. Phys. Let. **335,** 321-325

Baker J. (1997), J. Comput. Chem. **18,** 1079 - 1095

Baker J. et al (1996), J. Chem. Phys. **105,** 192 - 212

Boese et al (1999), Angew. Chem. Int. Ed **38,** 988

Bondi A. (1964), J. Phys. Chem. **68,** 441

Delley B. (1990), J. Chem. Phys. **92,** 508 - 517

Delley B. (2000), J. Chem. Phys. **113,** 7756 - 7764

Dunitz J. D. and Bernstein J. (1995), Disappearing Polymorphs, *Acc. Chem. Res.* **28** (4), 193-200

Elstner M. et al (2003), Journal of Molecular Structure (Theochem) **632**, 29-41

Elstner M. et al (2001), J. Chem. Phys **111,** 5149 - 5155

Foulkes W. M. C. et al (2001), Review of Modern Physics **73**, 33-83

Hahn T. (2002), International Tables for Crystallography: Volume A, Kluwer Academic Publishers

Halgren T. A. (1992), J. Am. Chem. Soc. **114**, 7827

Kresse G. and Hafner J. (1993), Phys. Rev. B **47,** 558

Kresse G. and Hafner J. (1994), Phys. Rev. B **49,** 14251

Kresse G. and Furthmüller J. (1996), Comput. Mat. Sci. **6**, 15

Kresse G. and Furthmüller J. (1996b), Phys. Rev. B **54**, 11169

Kresse G. and Joubert D. (1999), Physs Rev. B **59**, 1758

Kudin K. N. et al (2001), J. Chem. Phys. **114**, 2919 - 2923

Leach A. R. (2001 ), Molecular Modelling: Principles and Applications, Pearson Education Limited

Liu H. et al (2001), PROTEINS: Structure, Function, and Genetics **44**, 484 - 489

Lommerse J. P. M. et al (2000), Acta Cryst. B **56**, 697 - 714

McMullan R.K and Kvick A. (1992), Acta Cryst. **B 48,** 726-731

Mills R. L. et al (1986), J. Chem. Phys. **84,** 2837

Milman V. et al (2000), Int. J. Quantum Chem. **77**, 895 - 910

Motherwell W. D. S. et al (2002), Acta Cryst. B **58**, 647 - 661

Neumann et al (1999), J. Chem. Phys **110,** 516

Prager M et al (1991), J. Chem. Phys **95**, 2473

Press W. H. et al (2002), Numerical Recipes in C++, Cambridge University Press

Refson K. and Pawley G. S. (1986), Acta Cryst. **B42,** 402-410

Wimmer E. (2004), htW://www.accelas.com/technology/qm/erich

VASP (2004), *VASP the guide,* http://cms.mpi.univie.ac.at/vasp/vasp/vasp.html

Wu Q. and Yang W. (2002), J. Chem. Phys. **116**, 515 - 524

**Claims**

1. A method for the accurate determination of van der Waals parameters for high-precision determination of crystal structures and/or energies, comprising the steps of:

   - numerically simulating at least one crystal structure based on density functional theory (DFT) calculations combined with a potential energy term representing Van der Waals interactions;
   - providing reference data containing accurate information about said at least one crystal structure;
   - defining a deviation function (F) quantifying a deviation between said reference data and said at least one simulated crystal structure;
   - fitting at least one parameter of said van der Waals potential term in such a way as to minimize said deviation function (F); and
   - obtaining the accurate van der Waals parameters from the best fit.

2. A method according to claim 1, **characterized in that** said van der Waals potential term is defined as:

$$E_{disp} = \sum_{A,B} -f_{A,B}(r_{A,B})\frac{C_{6,A,B}}{r_{A,B}^6}$$

wherein $f_{A,B}(r_{A,B})$ is a damping function and the sum runs over all pairs of interacting atoms, and that said fitting step comprises fitting said damping function.

3. A method according to claim 2, **characterized in that** said damping function is defined as

$$f_{A,B}(r) = \left( 1 - \exp\left[ -c\left( \frac{r}{r_{A,B}} \right)^{\frac{3}{n}} \right] \right)^{2n}$$

and that said fitting step comprises fitting the parameter $r_{A,B}$ and/or the parameter n and/or the parameter c.

4. A method according to claim 2 or 3, **characterized in that** said fitting step furthermore comprises fitting said coefficient $C_{6,A,B}$.

5. A method according to any of the preceding claims, **characterized in that** said reference data are theoretical data obtained by Hartree-Fock calculations or Quantum Monte Carlo simulations.

6. A method according to any of the preceding claims, **characterized in that** said reference data are experimental low-temperature crystal structure data.

7. A method according to claim 6, **characterized in that** said crystal structure data are obtained by X-Ray or neutron scattering.

8. A method for the accurate determination of crystal structures and/or energies, comprising the steps of:

   - providing a rough estimate model of at least one crystal structure;
   - numerically simulating said at least one crystal structure based on density functional theory (DFT) calculations combined with a potential energy term representing Van der Waals interactions; and
   - obtaining said at least one crystal structure and/or its energy as a result of said numerical simulation,

   **characterized in that** said van der Waals potential term is obtained by the method according to any of claims 1 to 7.

9. A method according to claim 8, **characterized in that** a plurality of polymorphic crystal structures are determined and ranked according to their respective energies.

10. A method for the efficient numerical optimization of a molecular crystal structure using an advantageous crystal coordinate system, comprising the steps of:

   - providing a starting crystal lattice described by an initial coordinate system comprising lattice parameters and atomic positions in said crystal;
   - defining a so-called natural coordinate system and representing said starting crystal lattice in said natural coordinate system, said natural coordinate system comprising:

     • first coordinates describing symmetry-allowed lattice changes and defmed in such a way that changes of said first coordinates do not cause changes of the molecular geometry or a rotation of molecules with respect to each other and leave fractional coordinates of molecular centres constant;
     • second coordinates describing symmetry-allowed translations of said molecules in said crystal;
     • third coordinates describing symmetry-allowed rotations of said molecules in said crystal;
     • fourth coordinates describing symmetry-allowed changes of the molecular geometry;

   - transforming coordinates from said natural coordinate system to said initial coordinate system;
   - calculating the lattice energy and energy derivatives with respect to said initial coordinate system; and
   - transforming said energy derivatives from said initial coordinate system to said natural coordinate system,

   wherein a minimization algorithm is used for minimizing said lattice energy with respect to said natural coordinate system.

11. A method for the energy ranking of polymorphic crystal structures, comprising the steps of:

- providing rough estimate models of each of said crystal structures;
- numerically simulating each of said crystal structures based on density functional theory (DFT) calculations combined with a potential energy term representing Van der Waals interactions.
- obtaining accurate crystal structures and energies as a result of said numerical simulation; and
- ranking said accurate crystal structures according to their respective acccurate energies.

12. The method according to claim 11, **characterized in that** the crystals are crystals of pharmaceutical compounds.

13. The method according to claim 12, **characterized in that** it is applied to identify the most stable polymorphic form of a pharmaceutical compound.

14. The method according to any of claims 11 to 13, **characterized in that** it uses a method for the efficient optimization of the molecular crystal structure according to claim 10.

15. A computer program product comprising computer readable code for enabling a computer to perform a method according to any of the preceding claims when said code is executed.

**Fig. 1**

**Fig. 2**

**Fig. 3**

**Fig. 4**

**Fig. 5**

**Fig. 6**

**Fig. 7**

**Fig. 8**

Case A : Linear molecule          Case B : Non-linear molecule

**Fig. 9**

```
┌─────────────────────────┐
│   Choose a starting set of │
│    empirical parameters    │
└─────────────────────────┘
              │
              ▼
┌──────────────────────────────────────────────────┐
│              For each crystal structure:           │
│                                                    │
│   -  Minimize the lattice energy using the hybrid  │
│      method                                        │
│   -  Choose a set of points around the minimum     │
│      sufficient for the numerical determination of the │
│      second derivatives matrix                     │
│   -  Evaluate the DFT energy and gradients at the  │
│      minimum and the chosen points                 │
└──────────────────────────────────────────────────┘
              │
              ▼
┌──────────────────────────────────────────────────┐
│ Optimize the empirical parameters using the Powell algorithm. │
│                                                    │
│ (Use harmonic approximation and evaluate energies only at the │
│ chosen points. No recalculation of DFT energies and gradients.) │
└──────────────────────────────────────────────────┘
              │
              ▼
         ╱╲
        ╱    ╲
       ╱ Empirical ╲
      ╱ parameters  ╲─── No
      ╲ converged ? ╱
       ╲          ╱
        ╲        ╱
         ╲╱
          │ Yes
          ▼
      ┌────────┐
      │  Stop  │
      └────────┘
```

**Fig. 10**

Fig. 11

Fig. 12

rank 1

↓

experimental
structure at 15 K

rank 2

↓

similar to
rank 1

rank 3

↓

experimental
structure at 143 K

Fig. 13

DFT only

DFT & empirical potentials [Wu and Yang 2002]

DFT & empirical potentials [this work]

# Fig. 14

**Fig. 15**

**Fig. 16**

**European Patent Office**

# EUROPEAN SEARCH REPORT

Application Number

EP 04 29 0701

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | WU Q ET AL: "Empirical correction to density functional theory for van der Waals interactions" JOURNAL OF CHEMICAL PHYSICS, vol. 116, no. 2, 8 January 2002 (2002-01-08), pages 515-524, XP008042179 * the whole document * | 1-7,15 | G06F19/00 |
| A | PAYNE R S ET AL: "Examples of successful crystal structure prediction: Polymorphs of primidone and progesterone" INTERNATIONAL JOURNAL OF PHARMACEUTICS (AMSTERDAM), vol. 177, no. 2, 25 January 1999 (1999-01-25), pages 231-245, XP002317047 ISSN: 0378-5173 * page 231, left-hand column, line 1 - page 232, right-hand column, line 8 * * page 234, left-hand column, line 17 - page 244, left-hand column, line 7 * | 8,9, 11-13,15 | |
| A | KNAPMAN K.: "Polymorphic predictions" MODERN DRUG DISCOVERY, vol. 3, no. 2, March 2000 (2000-03), pages 53-54 AND 57, XP002317048 * the whole document * | 8,9, 11-13,15 | TECHNICAL FIELDS SEARCHED (Int.Cl.7) G06F |
| A | VERWER P ET AL: "COMPUTER SIMULATION TO PREDICT POSSIBLE CRYSTAL POLYMORPHS" REVIEWS IN COMPUTATIONAL CHEMISTRY, VCH, NEW YORK, NY, US, vol. 12, October 1998 (1998-10), pages 327-365, XP008042156 ISSN: 1069-3599 * the whole document * | | |

-/--

~~The present search report has been drawn up for all claims~~

| Place of search | Date of completion of the search | Examiner |
|---|---|---|
| The Hague | 10 February 2005 | Godzina, P |

EP 1 577 822 A1

European Patent Office

**EUROPEAN SEARCH REPORT**

Application Number

EP 04 29 0701

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | NEUMANN M A ET AL: "Recent advances in structure solution from powder diffraction data" INTERNATIONAL JOURNAL OF MODERN PHYSICS B WORLD SCIENTIFIC SINGAPORE, vol. 16, no. 1-2, 2002, pages 407-414, XP008042435 ISSN: 0217-9792 * abstract * * page 413, line 5 - page 414, line 2 * ----- | | |
| | | | TECHNICAL FIELDS SEARCHED (Int.Cl.7) |

~~The present search report has been drawn up for all claims~~

| Place of search | Date of completion of the search | Examiner |
|---|---|---|
| The Hague | 10 February 2005 | Godzina, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

European Patent
Office

**Application Number**

EP 04 29 0701

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-9, 11-13, 15

European Patent
Office

## LACK OF UNITY OF INVENTION
### SHEET B

Application Number

EP 04 29 0701

The Search Division considers that the present European patent application does not comply with the
requirements of unity of invention and relates to several inventions or groups of inventions, namely:

1. claims: 1-9, 11-13, 15

   Determining van der Waals parameters for the determination
   of crystal structures and/or their energies and for the
   ranking of energies of polymorphic crystal structures.
   ---

2. claims: 10, 14

   Numerically optimizing a molecular crystal structure using
   a crystal coordinate system.
   ---